# EUROPEAN PATENT SPECIFICATION

(11) **EP 1 185 306 B1**
(45) Date of publication and mention of the grant of the patent: **30.11.2005**
(21) Application number: 00941379.0
(22) Date of filing: 12.06.2000
(51) Int. Cl.: A61K 48/00, A61K 51/00, A01N 37/18, C12Q 1/68, C12P 21/06, C12N 15/00, C12N 11/00

(54) **USE OF VIRAL VECTORS AND CHARGED MOLECULES FOR GENE THERAPY**
VERWENDUNG VON VIRALEN VEKTOREN UND GELADENEN MOLEKÜLEN FÜR DIE GENTHERAPIE
UTILISATION DE VECTEURS VIRAUX ET DE MOLECULES CHARGEES DANS UNE THERAPIE GENIQUE

(30) Priority: 11.06.1999 US 138875 P
(43) Date of publication of application: 13.03.2002
(73) Proprietor: MediGene, Inc., San Diego, CA 92131 (US); UNIVERSITY OF BRITISH COLUMBIA, Vancouver, British Columbia V6T 1W5 (CA)
(72) Inventor: TUFARO, Francis, Vancouver, British Columbia V6S 1X4 (CA); YEUNG, Sonia, Vancouver, British Columbia V6M 1V7 (CA); HORSBURGH, Brian, Vancouver, British Columbia V5Y 3A8 (CA)
(74) Representative: Bösl, Raphael Konrad
(86) International application number: PCT/US2000/016167
(87) International publication number: WO 2000/076553

(56) References cited:
- US-A- 5 728 399
- US-A- 5 827 703
- US-A- 5 827 707
- DYER ANGELA P ET AL: "Dextran sulfate can act as an artificial receptor to mediate a type-specific herpes simplex virus infection via glycoprotein B." JOURNAL OF VIROLOGY, vol. 71, no. 1, 1997, pages 191-198, XP002197734 ISSN: 0022-538X
- BANFIELD BRUCE W ET AL: "Sequential isolation of proteoglycan synthesis mutants by using herpes simplex virus as a selective agent: Evidence for a proteoglycan-independent virus entry pathway." JOURNAL OF VIROLOGY, vol. 69, no. 6, 1995, pages 3290-3298, XP002197735 ISSN: 0022-538X
- ARCASOY S M ET AL: "POLYCATIONS INCREASE THE EFFICIENCY OF ADENOVIRUS-MEDIATED GENE TRANSFER TO EPITHELIAL AND ENDOTHELIAL CELLS IN VITRO" GENE THERAPY, MACMILLAN PRESS LTD., BASINGSTOKE, GB, vol. 4, 1997, pages 32-38, XP000917714 ISSN: 0969-7128
- KAPLAN J M ET AL: "POTENTIATION OF GENE TRANSFER TO THE MOUSE LUNG BY COMPLEXES OF ADENOVIRUS VECTOR AND POLYCATIONS IMPROVES THERAPEUTIC POTENTIAL" HUMAN GENE THERAPY, XX, XX, vol. 9, no. 10, 1 July 1998 (1998-07-01), pages 1469-1479, XP000858007 ISSN: 1043-0342
- YEUNG S N ET AL: "Efficient infection of mature skeletal muscle with herpes simplex virus vectors by using dextran sulfate as a co-receptor." GENE THERAPY, vol. 6, no. 9, September 1999 (1999-09), pages 1536-1544, XP002197736 ISSN: 0969-7128

## Description

### Background of the Invention

This invention relates to the use of viral vectors and charged molecules for gene therapy.

Skeletal muscle is an ideal seeding site for the treatment of primary myopathies or diseases requiring production of circulating proteins, because it is highly vascular and is an excellent secretory organ, with many accessible sites (Blau *et al.,* New Eng. J. Med. 333(23):1554-1556, 1995; van Deutekom *et al.,* Neuromuscular Disorders 8(3-4):135-148, 1998; Howell *et al.,* Human Gene Therapy 9(5):629-634, 1998; Isaka *et al.*, Nature Med. 2(4):418-423, 1996; Pauly *et al.,* Gene Therapy 5(4):473-480, 1998; Bohl *et al.,* Human Gene Therapy 8(2):195-204, 1997; Takeda, Nippon Rinsho - Japanese J. Clin. Med. 55(12):3114-3119, 1997, Tsurumi *et al.*, Circulation 96(9 Suppl.):II-382 8, 1997). Moreover, the post-mitotic nature and longevity of muscle fibers permits stable expression of transferred genes, even if they are not integrated into chromosomal DNA (Svensson *et al.,* Mol. Med. Today 2(4):166-172, 1996; van Deutekom *et al*., Mol. Med. Today 4(5):214-220, 1998). Also, high level gene expression in a relatively small number of muscle fibers may be adequate to treat inherited or acquired metabolic disorders, or to induce an immune response sufficient for vaccination (Davis *et al.,* Human Mol. Gen. 2(11):1847-.1851, 1993). Methods and compositions for introduction of a transgene into a plurality of mammalian tissues are already known in the art. For example, US 5,827,703 discloses a method of introducing an isolated transgene (optionally with regulatory sequences but without plasmid sequences) into a host cell. US 5,728;399 refers to a method of introducing polynucleotides into cells using polylysine, wherein the polynucleotide to be delivered may code for a viral protein. US 5,827,707 describes methods for producing minimal volume capsules containing retroviruses. Finally, Arcasoy et al. (Gene Ther. 1997 Jan;4(1):32-38) and Kaplan et al. (Hum Gene Ther. 1998 Jul 1;9.(10):1469-1479) disclose the use of recombinant adenovirus vectors for the delivery of various therapeutic genes, wherein polycations like poly-L-lysine are used for enhanced transduction of cells.

However, gene transfer to skeletal muscles has been hampered in part due to the inability of current generation vectors to infect a significant number of cells (Acsadi *et al.,* Nature 352(6338):815-818, 1991; Karpati *et al*., Muscle & Nerve 16(11):1141-1153; Smith *et al.,* Nature Genetics 5(4):397-402, 1093; Acsadi *et al.,* Human Mol. Gen. 3(4):579-584, 1994; Yang *et al.,* Proc. Natl. Acad. Sci. USA 91(l0):4407-4411, 1994; Dai *et al.,* Proc. Natl. Acad. Sci. USA 92(5):1401-1405, 1995; Huard *et al.,* Exp. & Mol. Path. 62(2):131-143, 1995; Mulligan, Science. 260(5110):926-932, 1993). Although adeno-associated virus (AAV) efficiently infects muscle and elicits sustained gene expression, its capacity for delivering and regulating large genes is limited. As for the large DNA viruses, such as Herpes Simplex virus (HSV) and adenovirus, muscle fibers exhibit a maturation-dependent loss of susceptibility to infection (Acsadi *et al.,* Human Mol. Gen. 3(4):579-584,1994; Huard *et al.* Human Gene Therapy 8(4):439-452, 1997; Feero *et al.,* Human Gene Therapy 8(4):371-380, 1997; Huard *et al.,* Neuromuscular Disorders 7(5):299-313, 1997; Huard *et al.,* J. Virol. 70(11):8117-8123, 1996; Huard *et al.,* Gene Therapy 2(6):385-392, 1995; Inui *et al.,* Brain & Dev. 18(5):357-361, 1996; Ragot *et al.,* Nature 361(6413):647-650, 1993; Quantin *et al.,* Proc. Natl. Acad. Sci. USA 89(7):2581 -2584, 1992; Vincent *et al.*, Nature Genetics 5(2):130.134, 1993). Previous studies of HSV infection in rodents show that the loss of infectivity may be due, at least in part, to the development of the basal lamina throughout the course of maturation, which may block the initial events in HSV infection (Huard *et al.*, J. Virol. 70(11):8117-8123,1996).

Cancer is another disease for which many therapies are being tested. One area of intense research in the cancer field is gene therapy. Cancer gene therapy suffers from many of the same problems as the muscle gene therapies described above. For example, the current vectors can not be delivered accurately to a sufficient number of cells to reduce tumor growth and increase patient survival.

To initiate infection, HSV attaches to cell surface glycosaminoglycans, such as heparan sulfate and dermatan sulfate (Spear *et al.,* Adv. Exp. Med. & Biol. 313:341-353,1992; Shieh *et al.,* 116(5):1273-1281, 1992; Fuller *et al.,* J. Virol. 66(8):5002-5012, 1992; Gruenheid *et al.*, J. Virol. 67(1):93-100,1993; Herold *et al.,* J. Gen. Virol. 75(6):1211-1222,1994; Banfield *et al.,* Virology 208(2):531-539, 1995; Williams *et al.,* J. Virol. 71(2):1375-1380, 1997), which stabilize the virus such that it can interact with secondary protein receptors required for entry into host cells (Terry-Allison *et al.*, J. Virol. 72(7):5802-5810, 1998; Geraghty *et al*., Science 280(5369):1618-1620, 1998; Montgomery *et al.,* Cell 87(3):427-436; 1996).

### Summary of the Invention

The invention provides uses of nucleic acid vectors for the preparation of pharmaceutical compositions for introducing vectors (*e.g.*, viral vectors, such as HSV vectors) into cells by co-administration of the vectors with a charged molecule (*e.g.*, a charged polysaccharide, such as a glycosaminoglycan or analog thereof). The pharmaceutical composition can be used to introduce genes into cells for use in gene therapy or vaccination.

Accordingly, the invention features uses of nucleic acid vectors for the preparation of pharmaceutical compositions for introducing a nucleic acid vector into a living cell, wherein said vector is a viral vector of the family Herpesviridae, and said vector is introduced into said cell by a method comprising contacting the cell with the vector and, either before, during, or after this contacting, contacting the cell with a liquid medium comprising a compound that, in the medium, is charged, non-cytotoxic, and capable of facilitating the uptake of the vector by the cell, wherein the cell is in a mammal, for example, a human patient. Vectors that can be used in the invention use glycosaminoglycans as receptors or co-receptors for entry into cells. Viral vectors-of the family Herpesviridae (*e.g.*, HSV-1, HSV-2, VZV, CMV, EBV, HHV6, and HHV7) are used. Preferably, the vectors are attenuated, and examples of attenuated viral vectors that can be used in the invention are provided below.

Molecules carrying either a negative or positive charge, that can be used in the invention include charged polysaccharides, such as glycosaminoglycans and analogs thereof, polylysine, acyclodextrin, and diethylaminoethane (DEAE). Examples of glycosaminoglycans and glycosaminoglycan analogs that can be used in the invention include, for example, dextran sulfate, dermatan sulfate, heparan sulfate, chondroitin sulfate, and keratin sulfate. An additional charged molecule that can be used in the invention is polyethylene glycol. As is discussed further below, the charged molecules can be administered prior to, or concurrent with, the vectors in the methods of the invention.

Cells that may be used in the invention include mature muscle cells, retinal cells, and cancer cells.

Conditions and diseases for which the pharmaceutical composition can be used include cancer, primary myopathies, and conditions and diseases that can be treated by production of a therapeutic product into circulation. Specific examples of these conditions and diseases, as well as genes that can be included in vectors to effect their treatment, such as genes encoding polypeptides (for example, growth factors, enzymes, anti-angiogenic polypeptides, and polypeptides that promote cell death), hormones, vaccine antigens, antisense molecules, and ribozymes, are described further below. In addition, the vector and charged molecule may be delivered to the subject locally or systemically.

The invention provides many advantages. For example, glycosaminoglycans and glycosaminoglycan analogs, such as dextran sulfate, are non-destructive, non-toxic, and limit the spread of viral vectors to other sites in the tissue. The uses of the invention, thus, represent an approach for targeted expression of genes in HSV vectors in desired cells or tissues by direct injection. Also, HSV is attractive as a gene delivery vector, because its large size allows for the delivery of several large genes at once, and it can be made relatively non-toxic (Huard *et al.,* Neuromuscular Disorders 7(5):299-313, 1997; Glorioso *et al.,* Annual Rev. Microbiol. 49:675-710, 1995). Moreover, HSV can be grown to high titers, can infect non-dividing cells efficiently (Lim *et al.,* Biotechniques 20(3):460-469, 1996), and can be controlled through the action of antiviral drugs, such as acyclovir, that inactivate virus replication (Evrard *et al.,* Cell Biol. & Toxic., 12(4-6):345-350, 1996; Black *et al*., Proc. Natl. Acad. Sci. USA 93(8):3525-3529, 1996; Hasegawa *et al.,* Am. J. Resp. Cell & Mol. Biol. 8(6):655-661,1993). Non-replicating HSV vectors also are relatively non-toxic, and thus can contribute to alleviation of the immunogenicity of foreign protein expression in tissues. Finally, as is noted above, skeletal muscle is an ideal site for the treatment of myopathies and other disorders, as it is highly vascular and is an excellent secretory organ, with many accessible sites.

Other features and advantages of the invention will be apparent from the following detailed description and the drawings.

### Brief Description of the Drawings

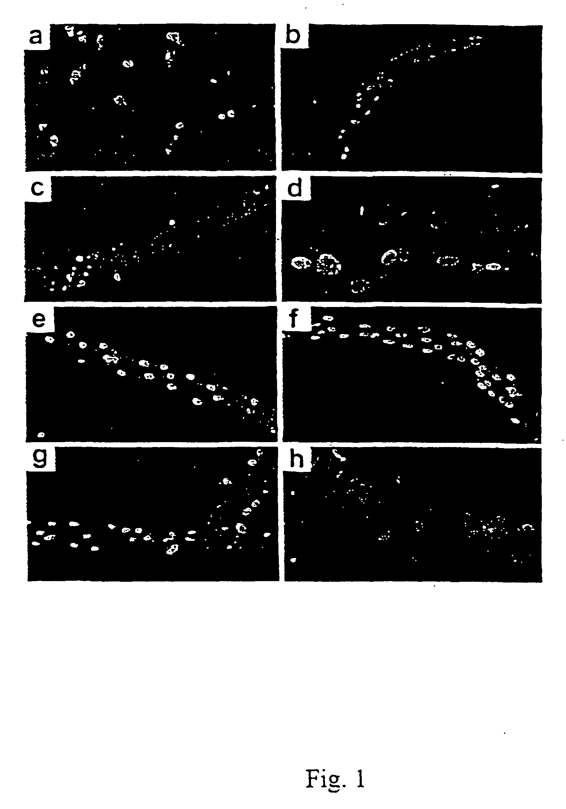

Fig. 1 is a photograph of HSV ICP4 Immunofluorescence showing infected nuclei of mature myofibers. Isolated myofibers infected with G207 were processed for indirect immunofluorescence using a mouse anti-ICP4 antibody (a) G207 only, mature myofiber, (b) G207 only, immature myofiber, (c) G207 only; (d) G207 + 0.33 mg/ml collagenase type IV; (e) G207 + 3 µg/ml dextran sulfate; (f) G207 + 10 µg/ml dextran sulfate; (g) G207 + 2 U/ml chondroitin ABC lyase; (h) G207 + 4 U/ml chondroitin ABC lyase. Magnification: a, b, c, e-g x 20; h x 40; d x 60. Images were captured by confocal microscopy: c-g.

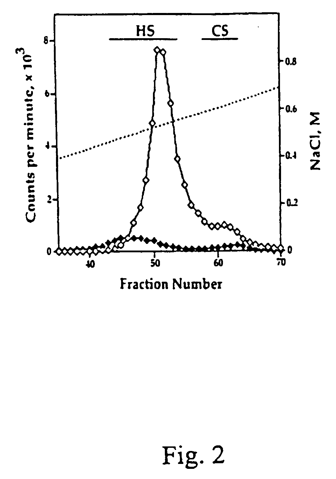

Fig. 2 is a graph showing anion-exchange HPLC of cell-associated glycosaminoglycans derived from myofibers belonging to different age-groups. Myofibers were labeled with [³⁵S] sulfate for 24 hours. The medium was removed, and the monolayers were washed extensively to remove any traces of medium from the cells. Glycosaminoglycans were isolated and fractionated by HPLC. HS, elution position of he CS, elution position of chondroitin sulfate. Open diamonds, myofibers isolated from 8-day old mice; closed diamonds, myofibers isolated from 2-month old mice. The dotted line represents the salt gradient used for elution.

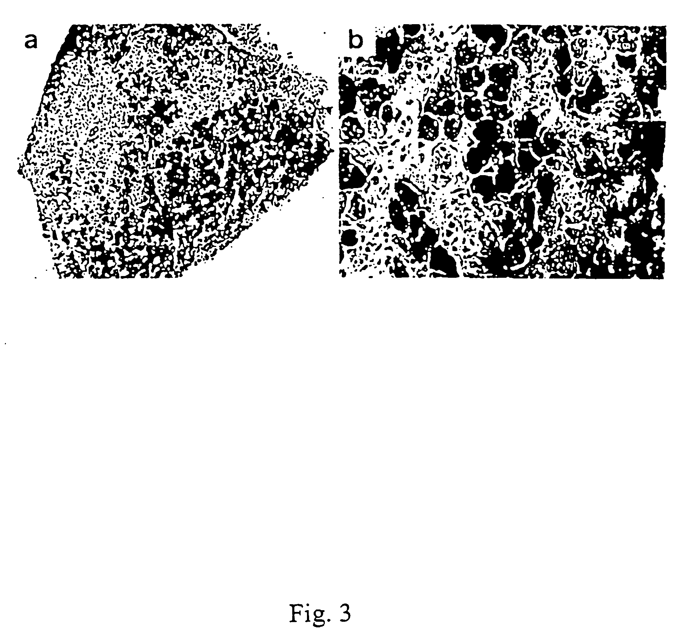

Fig. 3 is a photograph showing the results of *in vivo* injection of immature skeletal muscle with G207. Cryostat sections of immature mouse TA muscle were taken 3 days post-injection of HSV and stained histochemically for β-galactosidase. Gene transfer was carried out by intramuscular infection of G207 (1 x 10⁶ plaque forming units (pfu)) in a volume of 50 µl (a) & (b) G207 only Magnification: a x 10, b x 40.

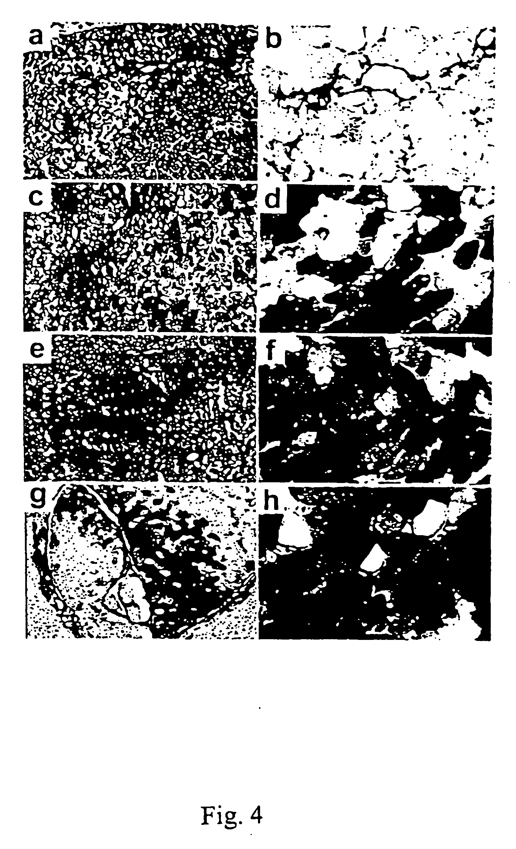

Fig. 4 is a photograph showing infection of mature skeletal muscle with G207- β-galactosidase gene transfer to skeletal muscle of adult mice. G207 (1 x 10⁶ pfu) and the.proposed treatments were co-injected into the tibialis anterior of 2-month old balb/c mice in a total infection volume of 50 µl. Frozen sections were cut and stained for β-galactosidase activity, (a) & (b) G207 only; (c) & (d) G207 + 0.33 mg/ml collagenase type IV; (e) & (f) G207 + 10 µg/ml dextran sulfate; (g) & (h) G207 + 2 U/ml chondroitin ABC lyase. Magnification: a, c, e, g x 20; b, d, f, h x 40.

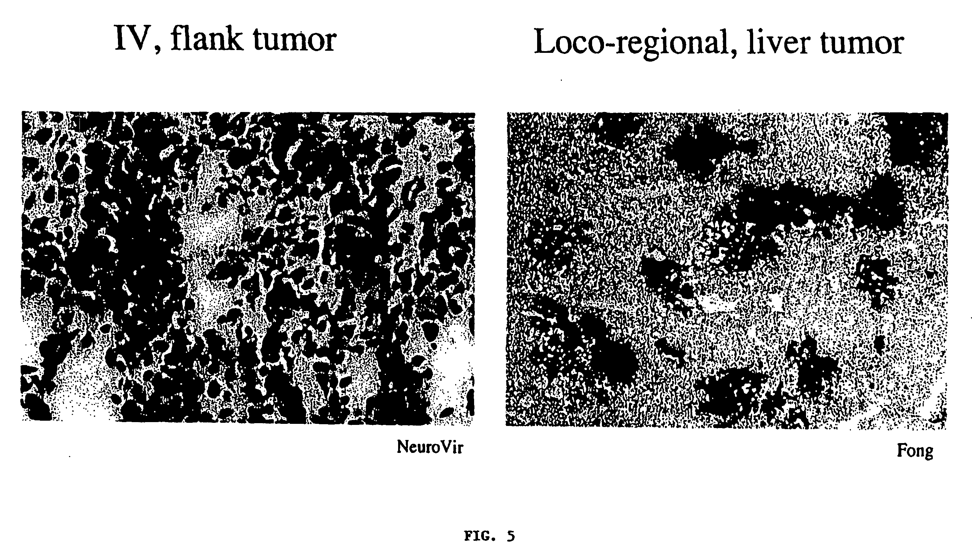

Fig. 5 is a set of photographs showing that HSV reaches tumor tissue after systemic delivery. HSV was administered to mice by tail vein, reaching a distant flank tumor (left panel), or locoregionally by portal vein, reaching a liver tumor (right panel). The tumor tissues were stained for β-galactosidase to detect cells infected with HSV.

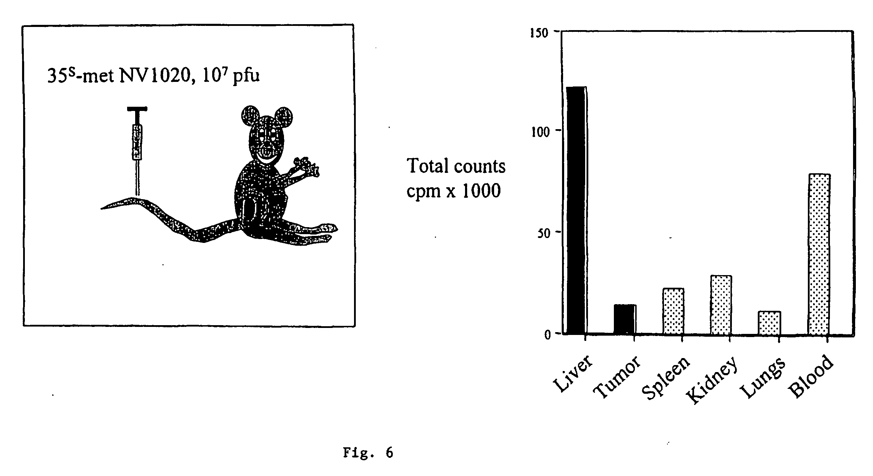

Fig. 6 is a schematic showing injection of labeled HSV in a mouse (right panel) and a graph of viral particle delivery to various tissues in mice with flank tumors. Mice were injected with 1 x 10⁷ pfu of ³⁵S methionine-labeled NV1020. Two hours later, the animals were sacrificed and their tissues were measured for the presence of viral particles.

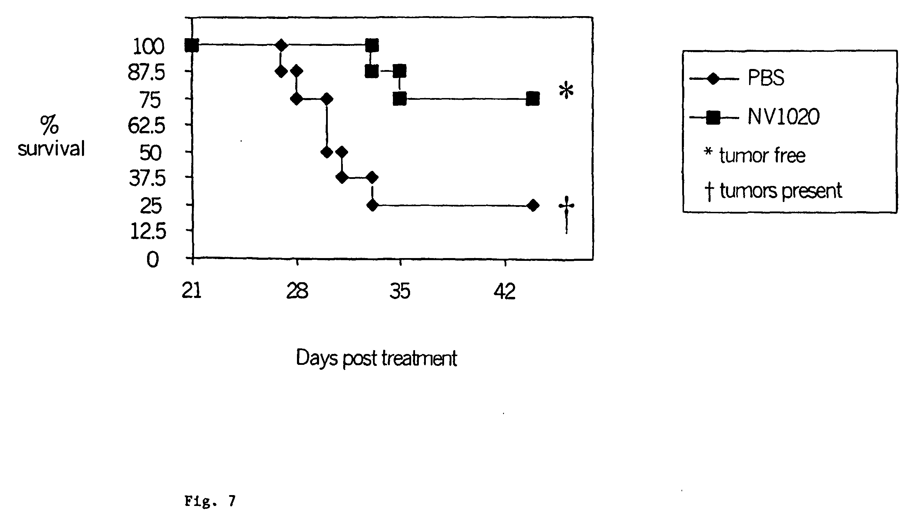

Fig. 7 is a graph showing the effect of systemic delivery of NV1020 (HSV) on mouse survival in a CT-26 liver metastatic cancer model. Mice with liver tumor nodules were systemically delivered 1 x 10⁷ pfu of NV1020 or PBS (control). The percent of surviving mice was measured over time.

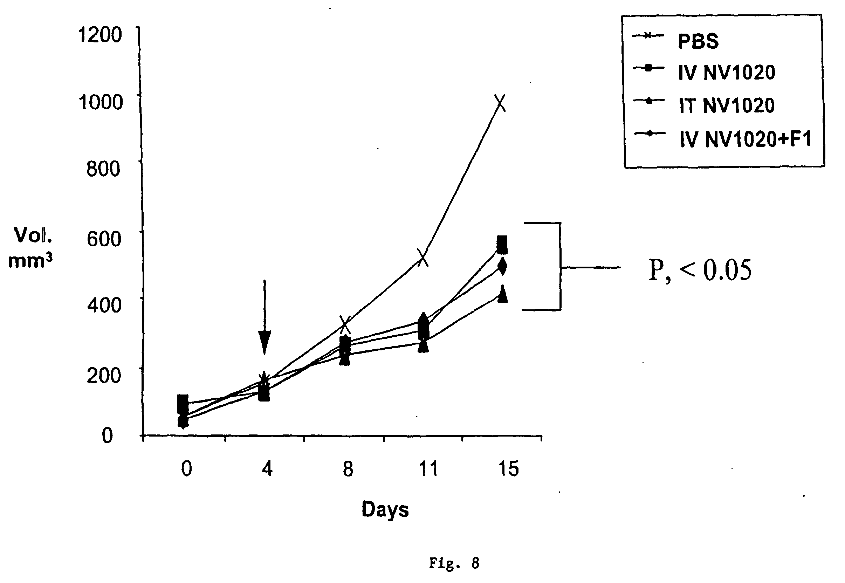

Fig. 8 is a graph showing the effect of intratumoral or systemic delivery of NV1020 or NV1020 plus F1 (dextran sulfate) on anti-tumor efficacy in a CT-26 flank tumor model. Mice with flank tumors were administered NV1020 either intratumorally (IT) or systemically (IV; with or without F1 (dextran sulfate)), by tail vein. Tumor growth rates were then assessed.

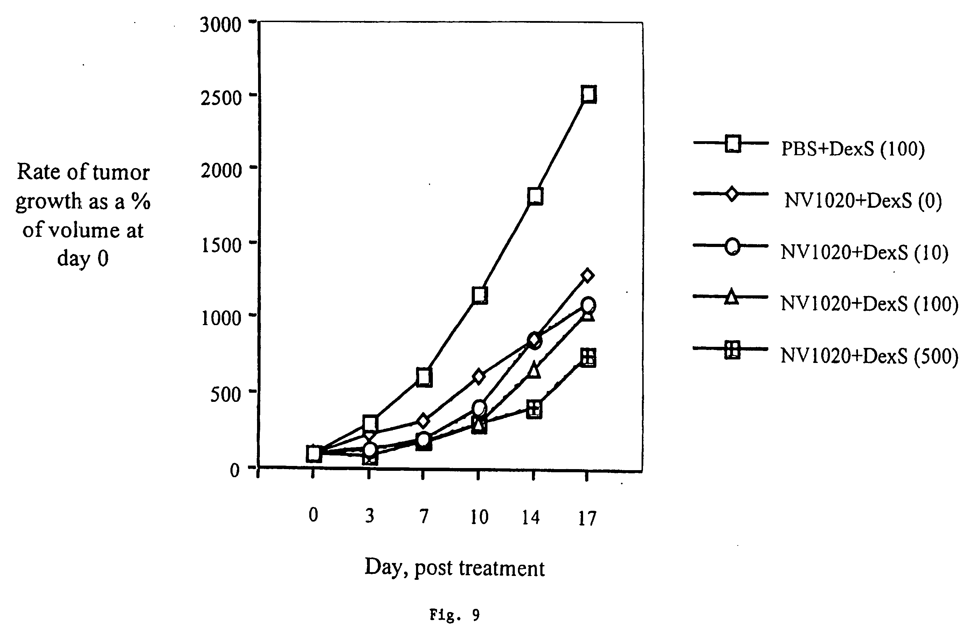

Fig. 9 is a graph showing the effect of dextran sulfate (DexS) dosage on anti-tumor efficacy. Mice with flank tumors were administered a dose of 1 x 10⁷ pfu of NV1020, 1 x 10⁷ pfu of NV1020 plus 10 µg/ml of dextran sulfate, 1 x 10⁷ pfu of NV1020 plus 100 µg/ml of dextran sulfate, 1 x 10⁷ pfu of NV 1020 plus 500 µg/ml of dextran sulfate, or PBS plus 100 µg/ml of dextran sulfate (control), at day 0, day 2, and day 4. Tumor growth rates were then assessed.

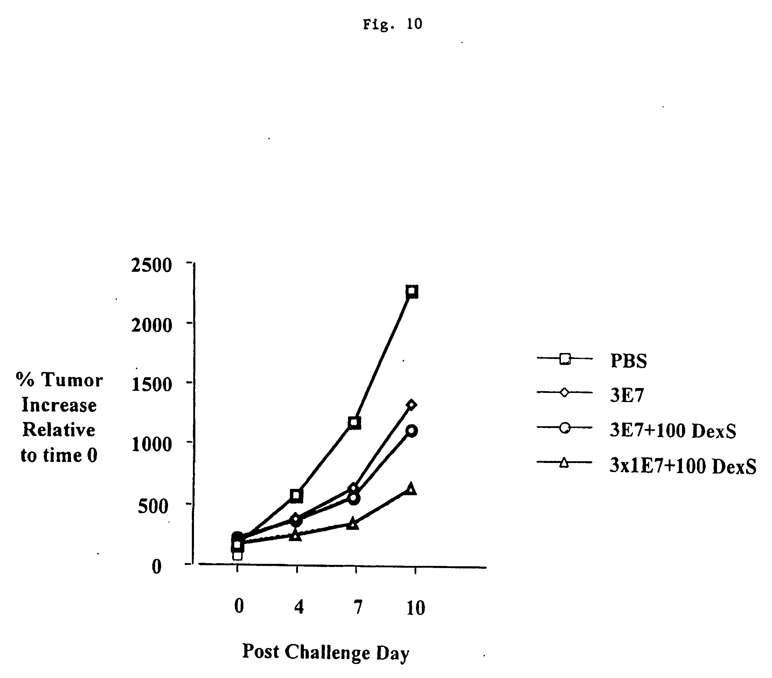

Fig. 10 is a graph illustrating the effects of multiple HSV plus dextran sulfate (DexS) dosing on anti-tumor efficacy in a CT-26 flank tumor model. Mice with flank tumors were administered one dose of 3 x 10⁷ pfu of NV1020, one dose of 3 x 10⁷ pfu of NV1020 plus 100 µg/ml of dextran sulfate, three doses of 1 x 10⁷ pfu of NV1020 plus 100 µg/ml of dextran sulfate, or PBS (control). Tumor growth rates were then assessed.

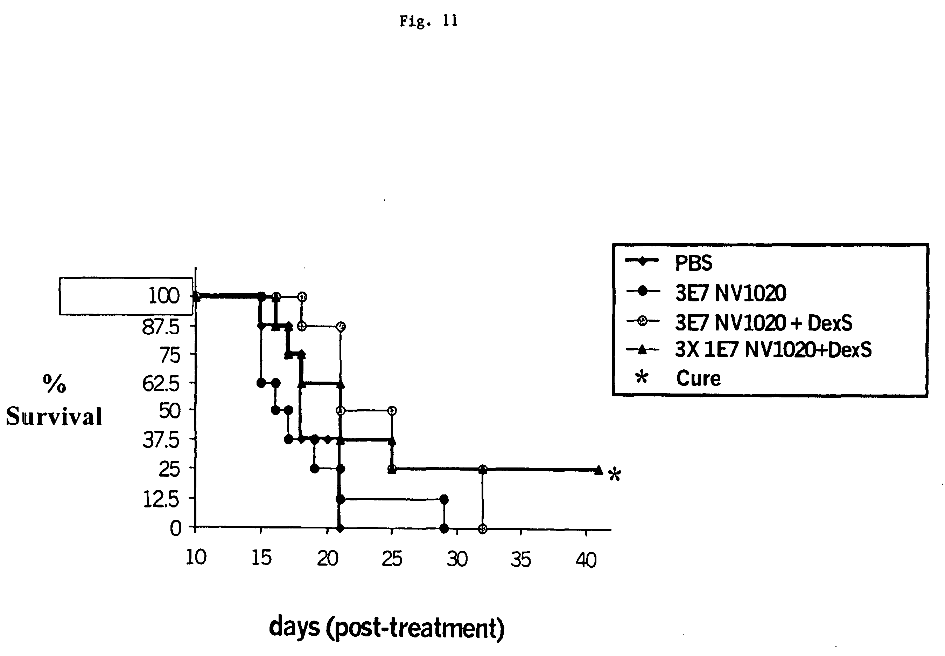

Fig. 11 is a graph showing the effect of multiple dosing of NV1020 plus dextran sulfate (DexS) on mouse survival in a CT-26 flank tumor model. Mice with flank tumors were administered one dose of 3 x 10⁷ pfu of NV1020 one dose of 3 x 10⁷ pfu of NV 1020 plus 100 µg/ml of dextran sulfate, three doses of 1 x 10⁷ pfu of NV1020 plus 100 µg/ml of dextran sulfate, or PBS (control). Mouse survival was then measured over time.

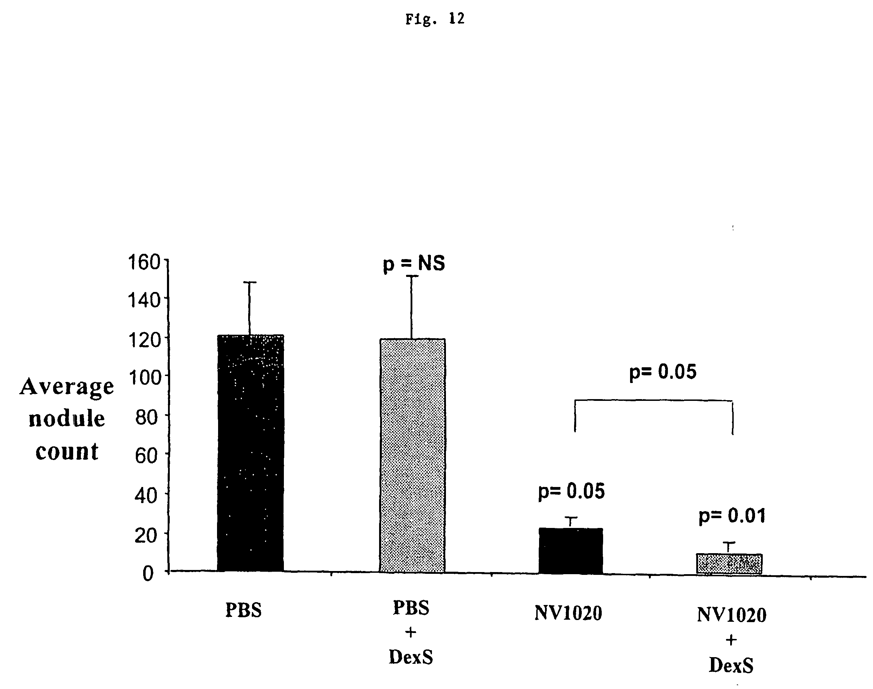

Fig. 12 is a graph showing the effect of dextran sulfate (DexS) on anti-tumor efficacy in a CT-26 liver metastatic model. Mice with metastasized tumors were administered 1 x 10⁷ pfu of NV1020, 1 x 10⁷ pfu of NV 1020 plus dextran sulfate (100 µg/ml), PBS only (control), or PBS plus dextran sulfate (100 µg/ml) (control). The number of tumor nodules was assessed 13 days later.

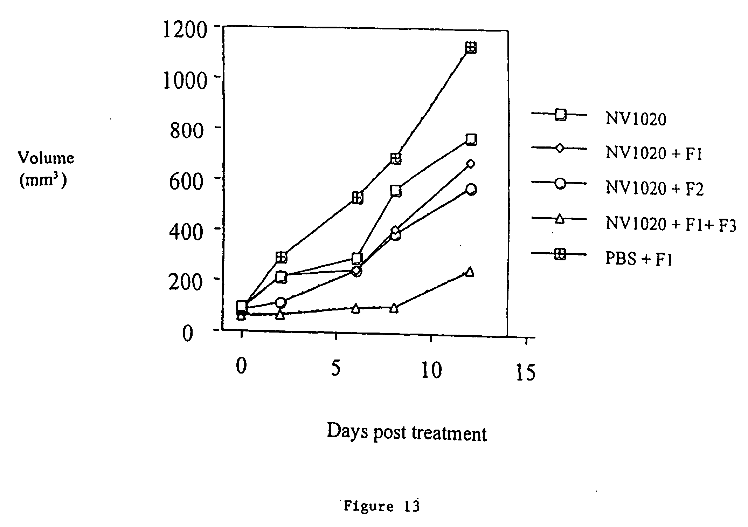

Fig. 13 is a graph illustrating the effects of dextran and acyclovir on anti-tumor efficacy in a CT-26 flank tumor model. Mice with flank tumors were administered a dose of 1 x 10⁷ pfu of NV 1020, 1 x 10⁷ pfu of NV1020 plus dextran sulfate (F1; 100 µg/ml) NV1020 plus dextran only (F2), 1 x 10⁷ pfu of NV1020 plus dextran sulfate and acyclovir (F3; 2 mg/ml), or PBS plus dextran sulfate (100 µg/ml) (control) at day 0, day 2, and day 4. Tumor growth rates were then assessed.

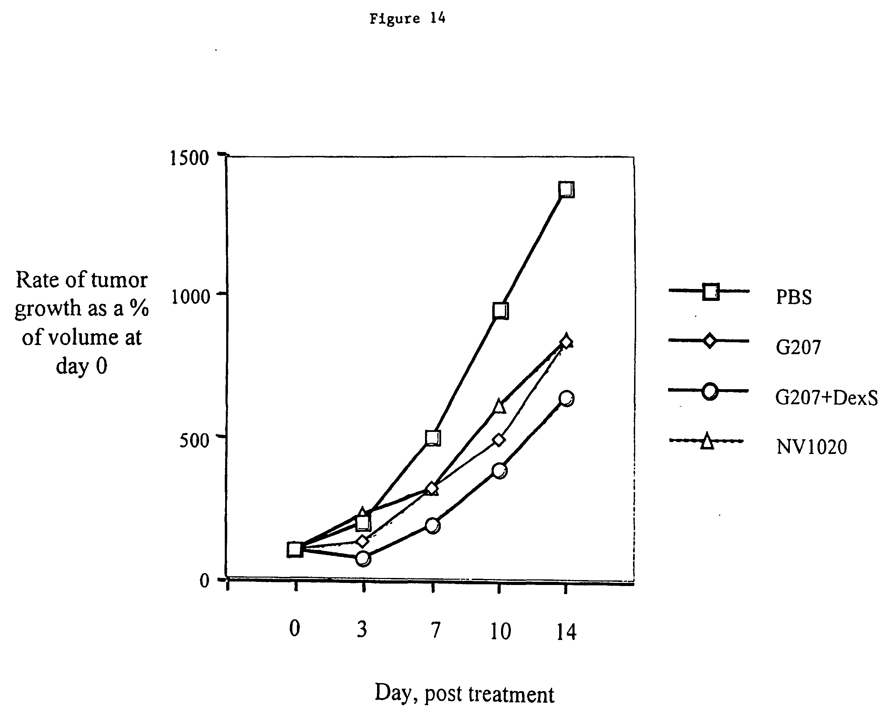

Fig. 14 is a graph showing the effect of G207 plus dextran sulfate (DexS) on anti-tumor efficacy. Mice with flank tumors were administered a dose of 1 x 10⁷ pfu of NV1020, 1 x 10⁷ pfu of G207, 1 x 10⁷ pfu of G207 plus dextran sulfate (100 µg/ml), or PBS (control) at day 0, day 2, and day 4. Tumor growth rates were then measured.

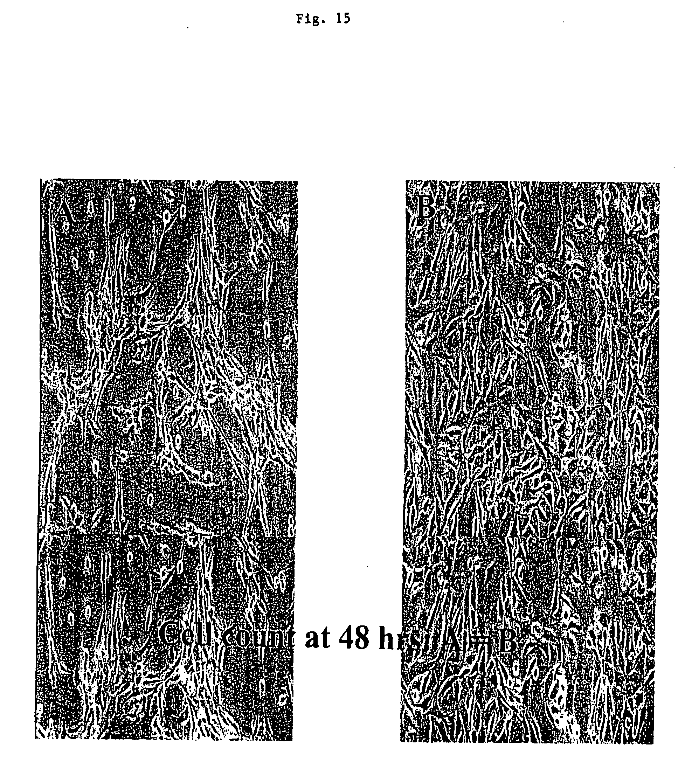

Fig. 15 is a set of photographs showing the effect of dextran sulfate (DexS) on CT-26 morphology and cell growth *in vitro.* No compound (panel A), or dextran sulfate (100 µg/ml; panel B) was added to CT-26 cells in culture. After 48 hours, cell numbers and morphology were examined.

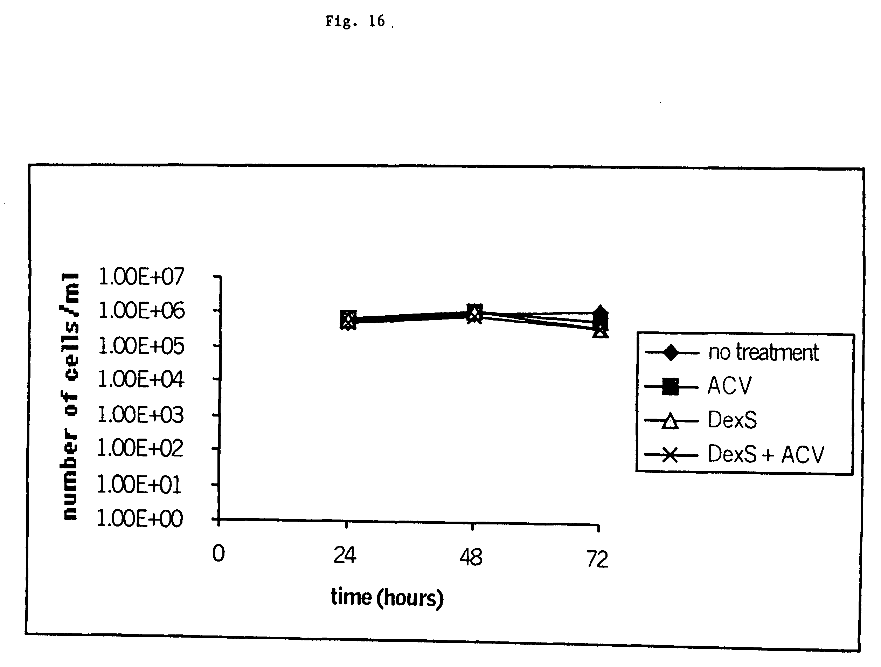

Fig. 16 is a graph showing the effects of dextran sulfate (DexS) and acyclovir (ACV) on CT-26 growth in culture. *In vitro* cultured CT-26 cells were administered dextran sulfate (100 µg/ml), acyclovir (2 mg/ml), both dextran sulfate and acyclovir, or were left untreated. Cell proliferation was measured over tune.

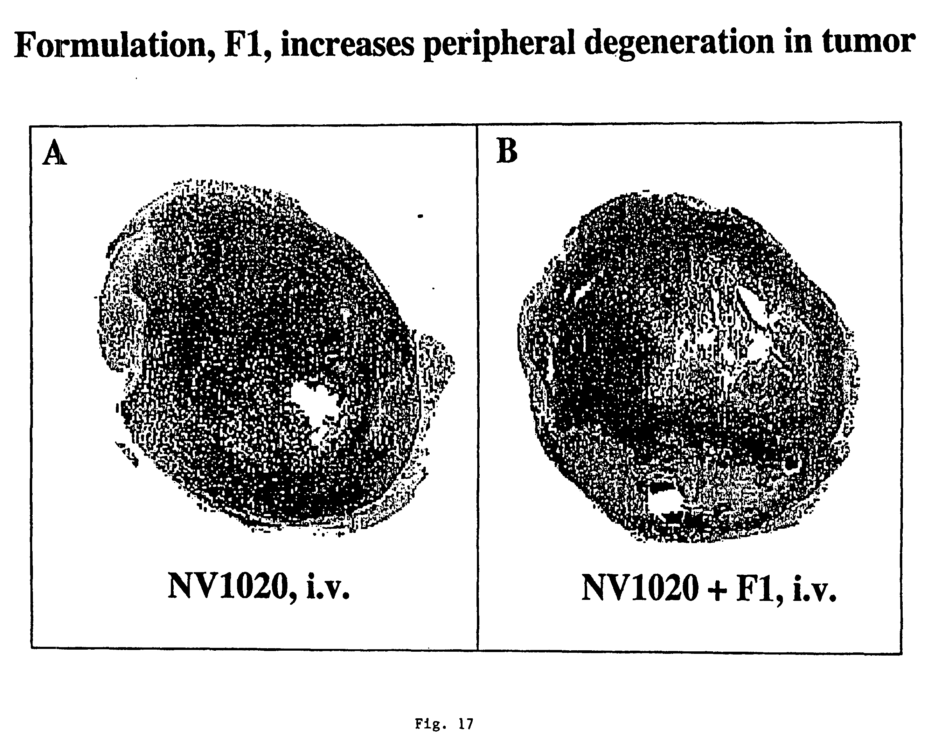

Fig. 17 is a set of photographs showing the effect of dextran sulfate on peripheral degeneration of flank tumors. Mice were administered 1 x 10⁷ pfu of NV1020 (panel A) or 1 x 10⁷ pfu of NV1020 plus dextran sulfate (F1) (panel B). The tumor was then removed and prepared for histochemical analysis is to evaluate tumor necrosis.

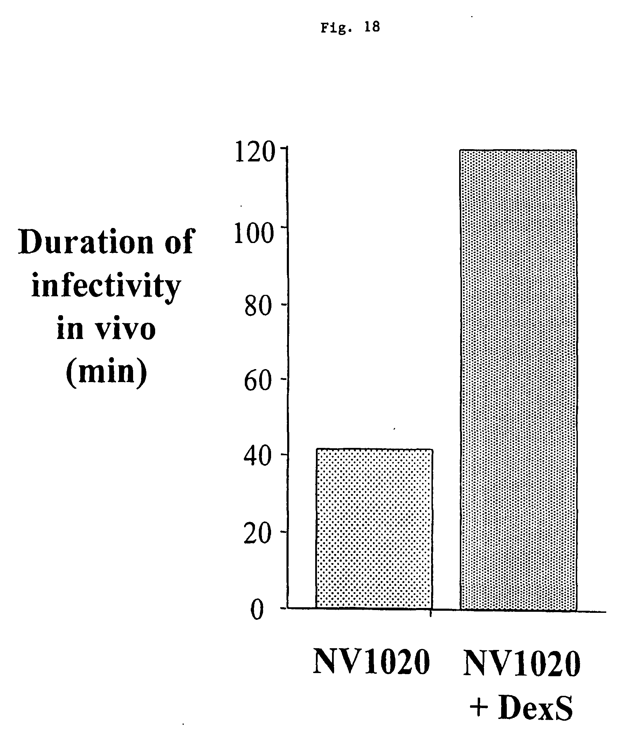

Fig. 18 is a graph showing the effect of dextran sulfate (DexS) on the bioavailability of virus *in vivo*. One x 10⁷ pfu of NV1020 or 1 x 10⁷ pfu of NV1020 plus dextran sulfate was administered by tail vein to mice. Groups of mice were then sacrificed at varying time points. Blood samples were removed and the serum was analyzed for infectious virus.

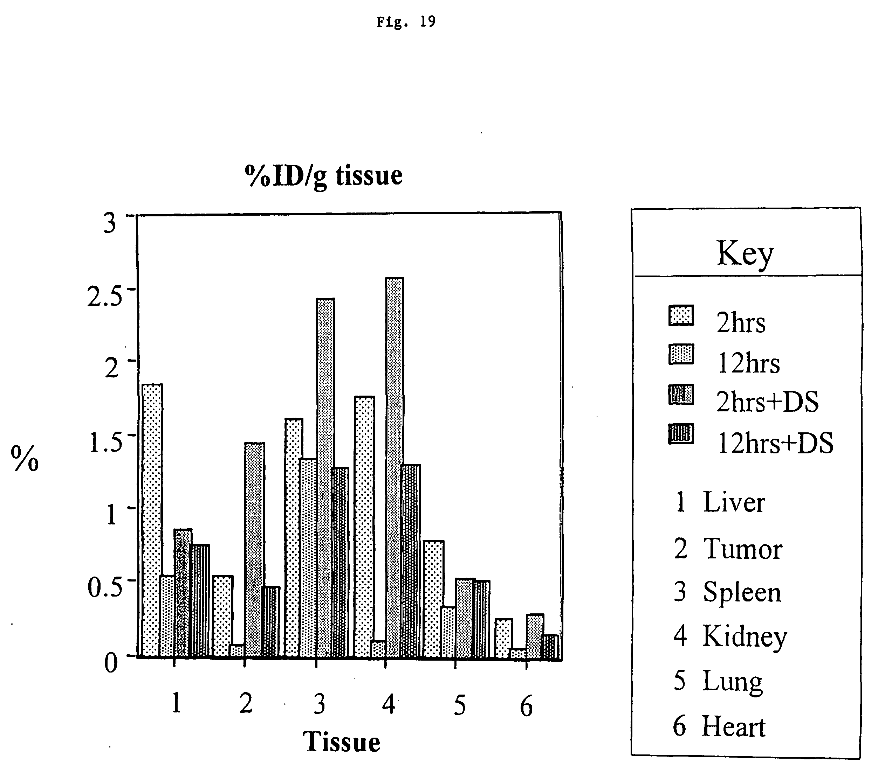

Fig. 19 is a graph demonstrating the effect of dextran sulfate (DexS) on the *in vivo* distribution of NV1020 in various tissues. One x 107 pfu of radioactive-labeled (³⁵S) NV 1020 or 1 x 10⁷ pfu of radioactive-labeled (³⁵S) NV1020 plus dextran sulfate (100 µg/ml) was administered to mice containing flank tumors by tail vein. After 2 hours or 12 hours, the animals were sacrificed, various organs were harvested, homogenized, and assessed for distribution of (³⁵S) NV1020.

### Detailed Description

The invention provides uses of nucleic acid vectors for the preparation of pharmaceutical compositions for introducing vectors (*e.g.*, viral vectors, such as HSV vectors) into cells, for example, mature skeletal muscle cells or tumor cells, by co-administering the viral vectors with a charged molecule, such as a charged polysaccharide, *e.g.*, a glycosaminoglycan or analog thereof. The pharmaceutical composition can be used to introduce genes into cells *in vivo* for the purpose of, for example, gene therapy or vaccination.

Vectors that can be used in the invention use glycosaminoglycans as receptors or co-receptors for entry into the cells. Viral vectors of the family Herpesviridae (*e.g.*, HSV-1, HSV-2, VZV, CMV, EBV, HHV-6, HHV-7, and HHV-8) are used.

In some cases, it is desirable that viral vectors used in the invention are attenuated or mutated, so that they do not replicate in or kill the cells into which they are introduced by, for example, inducing lysis or apoptosis of the cells. In other cases, for example, in tumor cell gene therapy, it is beneficial that the vectors can replicate in a cell and kill it. Numerous appropriate mutant viruses having these characteristics are known and can readily be adapted for use in the invention by those of ordinary skill in this art. For example, in the case of HSV, the vectors of Geller (U.S. Patent No. 5,501,979; WO 90/09441; American Type Culture Collection (ATCC), Rockville, Maryland, ATCC Accession Number 40544), Breakfield (EP 453,242-A1), Speck (WO 96/04395), Preston *et al.* (WO 96/04394), DeLuca (U.S. Patent No. 5,658,724), and Martuza (U.S. Patent No. 5,585,096) can be adapted for use in the methods of the invention. Specific examples of attenuated HSV mutants that can be used in the invention include NV1020 (described below), G207 (Yazaki et al., Cancer Res. 55(21):4752-4756, 1995), HF (ATCC VR-260), MacIntyre (ATCC VR-539), MP (ATCC VR-735);HSV-2 strains G (ATCC VR-724) and MS (ATCC VR-540); as well as mutants having mutations in one or more of the following genes: the immediate early genes ICP0, ICP4, ICP22, ICP27, and ICP47 (U.S. Patent No. 5,658,724); genes necessary for viral replication, UL9, UL5, UL42, DNA pol, and ICP8; the γ34.5 gene; the ribonucleotide reductase gene; the VP16 gene (*i.e.*, Vmw65, WO 91/02788; WO 96/04395; WO 96/04394); and the gH, gL, gD or gB genes (WO 92/05263, WO 94/21807, WO 94/03207).

Charged molecules that can be used in the invention include charged polysaccharides, such as glycosaminoglycans and analogs thereof, polylysine, acyclodextrin, and diethylaminoethane (DEAE). Examples of glycosaminoglycans and glycosaminoglycan analogs that can be used in the invention include, for example, dextran sulfate, dermatan sulfate, heparan sulfate, chondroitin sulfate, and keratin sulfate. An additional charged molecule that can be used in the invention is polyethylene glycol. As is discussed further below, the charged molecules can be administered prior to, or concurrent with, the vectors in the uses of the invention.

Conditions that can be treated with the uses of the invention include cancer, primary myopathies, as well as conditions that can be treated by the production of circulating proteins. Thus, vectors in the uses of the invention can include one or more genes encoding one or more therapeutic gene products, such as a polypeptide, for example, a growth factor, an enzyme, a polypeptide that promotes cell death, an anti-angiogenic polypeptide, or an immunomodulatory protein, a hormone, an antisense RNA molecule, or a ribozyme (see below), expression of which will alleviate or prevent a symptom of a condition or disease. Alternatively, the gene can encode a vaccine antigen and the uses of the invention, thus, can be used to induce a prophylactic or therapeutic immune response, for example, to an undesired pathogen or cell type, such as a cancer cell.

Specific examples of conditions that can be treated using the invention (as well as corresponding genes to be included in vectors for treating the conditions) are as follows: restenosis (β-ARKct (laccarino *et al.,* Proc. Natl. Acad. Sci. USA 96(7):3945-3954, 1999); fibroblast growth factor receptor (Yukavsra *et al.*, Atherosclerosis 141(1):125-132, 1998)); laryngeal paralysis and muscle atrophy, by enhancement of nerve sprouting and muscle re-innervation (insulin-like growth factor-1 (IGF-1) (Shiotani *et al.,* Archives Otolaryngology.125(S):SSS-56U, 1999)); mucopolysaccharidosis type VII (β-glucuronidase (Daly *et al.*, Human Gene Therapy 10(1):85-94, 1999); lunb-girdle muscular dystrophies 2C-F (δ-sarcoglycan (Greelish *et al.*, Nature Medicine 5(4):439-443, 1999)); fibrotic diseases, such as glomerulonephritis and glomerulosclerosis (transforming growth factor-β type IT receptor-IgG Fc chimera (Isaka *et al.*, Kidney Intl. 55(2):740-741, 1999)); mucopolysaccharidosis type VI (N-acetylgalactosamine 4-sulfatase (Yogalingam *et al.*, DNA & Cell Biol. 18(3):187-195, 1999)); motor neuron diseases (neurotrophin-3 and other neurotrophic factors, such as CNTF, BDNF and IGF-1 (Haase *et al.*, J. Neuro. Sci. 160(Suppl.1):S97-105, 1998)); hypertension (angiotensin II type I receptor antisense (Gelband *et al.*, Hypertension 33(1):360-365, 1999)); atherosclerosis and hypercholesterolemia (apolipoprotein AI and lecithin-cholesterol acyltransferase (Fan *et al.*, Gene Therapy 5(10):1434-1440, 1998)); induction of an alloimmune response (donor MHC class I (Zhai *et al.*, Transplant Immunology 6(3):169-175, 1998)); hemophilia (Factor VIII, Factor IX (Herzog ef *al.*, Nature Medicine 5(1):56-63, 1999; Herzog *et al.*, Cur. Opin. Hemat. 5(5):321-326,1998)); loss of skeletal muscle function in aging (IGF-1 (Barton-Davis *et al.*, Proc. Natl. Acad. Sci. USA 95(26):15603-15607, 1998)); liver enzyme deficiencies (phenylalanine hydroxylase (Harding *et al.*, Gene Therapy 5(5):677-683, 1998)); non-insulin dependent diabetes mellitus (GLUT4 (Galuska *et al.*, Adv. Exp. Med. & Biol. 441:73-85, 1998)); glycogen storage disease (acid alpha-glucosidase (Nicolino *et al.*, Human Mol. Gen. 7(11):1 695-1702, 1998)); muscular dystrophy (dystrophin (Baranov *et al.*, Genetika 34(7):876-882, 1998; utrophin; Rafael *et al.*, Nature Genetics 19(1):79-82, 1998)); tumor and metastasis suppression, vaccine adjuvant, and pathogen defense (interleukin-12 (Lee *et al.*, Human Gene Therapy 9(4):457-465, 1998)); and acute limb ischemia (vascular endothelial growth factor (Tsurumi *et al.*, Circulation 96(Suppl. 9):II-382-8, 1997)). Additional therapeutic products that can be produced using the invention include, for example, growth hormone, erythropoietin, and insulin, immunomodulatory proteins, antiangiogenic proteins, cytokines, and polypeptides involved in cell death.

As is noted above, the therapeutic product encoded by a gene in a vector used in the invention can also be an RNA molecule, such as an antisense RNA molecule that, by hybridization interactions, can be used to block expression of a cellular or pathogen mRNA. Alternatively, the RNA molecule can be a ribozyme (*e.g.*, a hammerhead or a hairpin-based ribozyme) designed either to repair a defective cellular RNA or to destroy an undesired cellular or pathogen-encoded RNA (see, *e.g.*, Sullenger, Chem. Biol. 2(5):249-253, 1995; Czubayko *et al*., Gene Ther. 4(9):943-949,1997; Rossi, Ciba Found. Symp. 209:195-204, 1997; James *et al.,* Blood 91(2):371-382, 1998; Sullenger, Cytokines Mol. Ther. 2(3):201-205, 1996; Hampel, Prog. Nucleic Acid Res. Mol. Bio. 58:1-39, 1998; Curcio *et al.,* Pharmacol. Ther. 74(3):317-332, 1997).

Genes can be inserted into vectors used in the invention using standard methods (see, *e.g.*, Ausubel *et al., Current Protocols in Molecular Biology,* John Wiley & Sons, New York, NY, 1998). The genes can be inserted so that they are under the control of vector regulatory sequences. Alternatively, the genes can be inserted as part of an expression cassette that includes regulatory elements, such as promoters or enhancers. Appropriate regulatory elements can be selected by one of ordinary skill in this art based on, for example, the desired tissue-specificity and level of expression. For example, a tissue- or cell type-specific (*e.g.*, muscle-specific or a tissue in which a tumor occurs) promoter can be used to limit expression of a gene product to a specific tissue or cell type. In addition to using tissue-specific promoters, local administration of the vector and/or charged molecule can be used to achieve localized expression.

Examples of non-tissue-specific promoters that can be used in the invention include the early Cytomegalovirus (CMV) promoter (U.S. Patent No. 4,168,062) and the Rous Sarcoma Virus promoter (Norton *et al.,* Molec. Cell Biol. 5:281, 1985). Also, HSV promoters, such as HSV-1 IE and IE4/5 promoters, can be used. An example of a tissue-specific promoter that can be used in the invention is the desmin promoter, which is specific for muscle cells (Li *et al.,* Gene 78:243, 1989; Li *et al*., J. Biol. Chem. 266:6562, 1991). Other muscle-specific promoters are known in the art, and can readily be adapted for use in the invention.

The vectors and charged molecules can be administered to a patient (*e.g.*, a human patient) according to the uses of the invention by, for example, direct injection into a tissue, for example, a muscle or a tissue in which a tumor is present, or by surgical methods. Alternatively, administration of one or both of these agents can be parenteral, intravenous, subcutaneous intraperitoneal, intradermal, or intraepidermal route, or *via* a mucosal surface, *e.g.*, an ocular, intranasal, pulmonary, oral, intestinal, rectal, vaginal, or urinary tract surface.

Any of a number of well known formulations for introducing vectors into cells in mammals can be used in the invention (see, *e.g.*, *Remington's Pharmaceutical Sciences* (18^{th} edition), ed., A. Gennaro, 1990, Mack Publishing Co., Easton, PA). For example, the vectors can be used in a naked form, free of any packaging or delivery vehicle. The vectors (as well as the charged molecules) can be simply diluted in a physiologically acceptable solution, such as sterile saline or sterile buffered saline, with or without a carrier.

The amount of vector to be administered depends, *e.g.*, on the specific goal to be achieved, the strength of any promoter used in the vector the condition of the mammal intended for administration (*e.g.*, the weight, age and general health of the mammal), the mode of administration, and the type of formulation. In general, a therapeutically or prophylactically effective dose of, *e.g.*, from about 1 ng to about 1 mg, preferably, from about 10 µg to about 800 µg, is administered to human adults.

The amount of charged molecule to be administered can be determined by one of skill in this art, and can be, for example, from about 1 ng/ml to about 100 µg/ml, but, preferably, is less than 10 µg/ml. Administration of both the vector and the charged molecule can be achieved in a single dose or repeated at intervals. Also, the charged molecule can be administered concurrently with or prior to (*e.g.*, up to five hours, such as three hours) the vector.

The uses of the invention are based on our discovery, which is described further below, that glycosaminoglycan synthesis is down-regulated during murine skeletal muscle maturation. This could account for the loss of HSV infectivity in maturing murine skeletal muscle, because heparan sulfate acts as a co-receptor for attachment of HSV to cells (Montgomery *et al.,* Cell 87(3):427-436, 1996; Geraghty *et al.,* Science 280(5369): 1618-1620, 1998; Whitbeck *et al*., J. Virol. 71(8):6083-6093, 1997). To test whether secondary HSV receptors were present, myofibers were treated with a variety of enzymes, including collagenase type IV and chondroitin ABC lyase. Both of these treatments enhanced HSV infection, which suggests that virus receptors were present, but not readily accessible to the virus in the intact myofiber. Surprisingly, we also found that infectivity of HSV-1, but not HSV type 2 (HSV-2), could be restored by exposing myoftbers to low concentrations of the glycosaminoglycan analog dextran sulfate. Dextran sulfate has been shown previously to promote HSV-1, but not HSV-2, infection in the absence of heparan sulfate. This supports the hypothesis that a lack of accessible heparan sulfate is responsible for the resistance of mature myofibers to HSV-1 infection. Taken together, these results show that the basal lamina is not an absolute block to infection, and that dextran sulfate can be used as a surrogate co-receptor for the nondestructive targeting of HSV-1 to mature skeletal muscle. These findings, which are described in detail below, greatly expand the usefulness of HSV as a gene therapy vector for the treatment of inherited and acquired diseases.

We have also discovered that the infection of tumor cells by HSV is increased through co-administration of a charged molecule, for example, a glycosaminoglycan or glycosaminoglycan analog. These results, further described below, again bolster the use of HSV as a gene therapy vector in treating cancer or other cell proliferation diseases or conditions.

### Results

### Mature myofibers are refractory to infection

It has been shown previously that HSV vectors infect newborn muscle fibers *in vitro,* but not those isolated from older animals (Huard *et al.*, Human Gene Therapy 8(4):439-452, 1997; Feero *et. al.*, Human Gene Therapy 8(4):371-380, 1997; Huard *et al.*, Neuromuscular Disorders.7(5):299-.313, 1997; Huard *et al.,* J. Virol. 7(11):8117-8123, 1996). To investigate the underlying basis for the maturation-dependent loss of infection, single muscle fibers were established in culture, and exposed to G207, which is an attenuated replication-defective HSV-1 vector that expresses β-galactosidase following infection (Yazaki *et al.*, Cancer Res. 55(21):4752-4756, 1995; Mineta *et al.*, Nature Med. 1(9):938-943, 1995). In these assays, newborn myofibers were completely susceptible to infection, whereas only 6% of mature myofibers were infected at this concentration of virus (Table 1 and Fig. 1, a and b). Thus, these results were consistent with previous studies showing a maturation-dependent loss of susceptibility to HSV infection (Feero *et al*., Human Gene Therapy 8(4):371-380,1997; Huard *et al.*, J. Virol, 70(11):8117-8123 1996).

**Table 1**

| Number of myofibers isolated from mature mouse EDL muscle that express *lac* Z following inoculation with G207 and proposed treatments | | |
|---|---|---|
| **Treatment** | **Total # of fibers** | **% positive** |
| G207 only | 88 | 6 |
| 0.02 mg/ml collagenase type IV | 68 | 7 |
| 0.20 mg/ml collagenase type IV | 81 | 31 |
| 0.33 mg/ml collagenase type IV | 77 | 97 |
| 0.66 mg/ml collagenase type IV | 67 | 0* |
| 0.3 µg/ml dextran sulfate | 78 | 6 |
| 3.0 µg/ml dextran sulfate | 75 | 7 |
| 10 µg/ml dextran sulfate | 82 | 99 |
| 2U/mIchondroitinABC!yase | 49 | 100 |
| 4 U/ml chondroitin ABC lyase | 44 | 30 |
| 6 U/ml chondroitin ABC lyase | 43 | 0 |
| 1-6U/ml heparitinase | 39 | 0 |
| PEG | 33 | 6 |

| | | |
|---|---|---|
| * 0.66 mg/ml collagenase type IV was toxic to isolated myofibers | | |

### Approaches to rescue adult skeletal muscle infectivity

Previous studies have suggested that basal lamina formation during maturation may act as a physical barrier to HSV infection, thereby preventing interaction of the virus with the receptors required for infectivity. To test this, isolated myofibers were exposed to G207 following treatment with collagenase type N, which liberates peptides from collagen thereby degrading the basal lamina (Fig. 1). Indirect immunofluorescence of a nuclear HSV protein, ICP4, revealed that partial destruction of the basal lamina in this manner stimulated HSV infection (Fig. 1, d). The effect was concentration dependent such that an increase in collagenase type IV correlated with an increase in HSV infection. Toxicity occurred at 0.66 mg/ml as indicated by myofiber hypercontraction during the 30 minute preincubation period (Table 1). In a second approach, chondroitin ABC lyase, which degrades a broad range of chondroitin sulfate moieties, was tested for its ability to enhance susceptibility to HSV infection (Fig. 1, g and h). This treatment strongly enhanced infection, whereas treatment with heparitinase did not (Table 1). Thus, partial destruction of the basal lamina with specific enzymes allowed for the attachment and entry of HSV into the mature muscle fiber, which suggests that virus secondary receptors were present but not accessible in the context of the mature myofiber.

### Analysis of cell surface glycosaminoglycans

HSV infects cells by attaching to cell surface heparan sulfate-like moieties followed by interaction with secondary protein receptors (Spear *et al*., Adv. Exp. Med. & Biol. 313:341-353, 1992; Gruenheid *et al.,* J. Virol. 67(1):93-100, 1993; Geraghty *et al*., Science 280(5369):1618-1620, 1998; Montgomery *et al*., Cell 87(3):427-436,1996). Although not strictly required, cell surface heparan sulfate increases the efficiency of HSV infection by two orders of magnitude in most cells tested (Banfield *et al.,* J. Virol. 69(9):3290-3298, 1995). To investigate whether glycosaminoglycan expression was altered in adult versus newborn muscle fibers, radiolabeled glycosaminoglycans were isolated from muscle fiber cultures and analyzed by anion-exchange HPLC (Fig. 2). Newborn muscle fibers expressed significant amounts of heparan sulfate and chondroitin sulfate glycosaminoglycans. By contrast, glycosaminoglycan synthesis was significantly reduced in adult myofibers during steady-state labeling, and the residual heparan sulfate synthesized was relatively under-sulfated compared with newborn myofibers (Fig. 2).

### Dextran sulfate restores HSV infection in mature myofibers

The data so far indicated that one or more components of the basal lamina present in mature myofibers inhibited HSV infection. Moreover, heparan sulfate biosynthesis was reduced compared with immature myofibers, which could account for all or part of the loss of susceptibility to HSV infection. It has been shown previously that cells devoid of heparan sulfate biosynthesis can be infected with HSV-1, but not HSV-2, if a low concentration of dextran sulfate is added to the cells either prior to or during infection (Dyer *et al.,* J. Virol. 71(1):191-198, 1997). By contrast, dextran sulfate is a potent inhibitor of HSV infection if the target cells express significant amounts of heparan sulfate. When dextran sulfate was added to mature myofibers in culture, HSV-1 infection was significantly enhanced (Fig. 1, e and f, and Table 1). Moreover, this effect was specific for HSV-1, which is consistent with the hypothesis that the lack of mature myofiber infection was due, at least in part, to a lack of accessible heparan sulfate moieties on the cell surface (Table 2).

**Table 2**

| Dextran sulfate stimulation of mature myofibers with G207 (HSV-1) vs. L1BRI (HSV-2) | | |
|---|---|---|
| **Treatment** | **Virus** | **β-galactosidase expression** |
| No treatment | G207 | negative |
| | L1BR1 | negative |
| 10 µg/ml dextran sulfate | G207 | positive |
| | L1BR1 | negative |

To test whether there was an additional block in the post-attachment fusion of HSV with the plasma membrane, isolated mature myofibers were exposed to the fusogenic agent polyethylene-glycol (PEG) prior to challenge with G207. PEG-induced fusion did not after adult myofiber infectivity, suggesting that the block to HSV infection occurred at the level of viral attachment (Table 1).

### Infection of skeletal muscle

To establish that immature myofibers were susceptible to G207 infection *in vivo,* 10⁶ plaque forming units (pfu) were injected directly into the tibialis anterior (TA) muscle of an 8-day old balb/c mouse. Injected muscles were removed three days post-injection, sectioned, and analyzed histochemically for the expression of β-galaciosidase (Fig. 3). High levels of transgene expression were detected in the injected area. HSV injected myofibers were also found away from the site of injection, which suggests that there was considerable spread of the vector.

To test whether the three treatments that enhanced infection *in vitro* worked in the adult animal, mice were injected with 1 x 10⁶ pfu of G207 in the tibialis anterior (TA) muscle along with either chondroitin ABC lyase, collagenase type IV, or dextran sulfate. In all instances, the *in vivo* results were consistent with the observations made *in vitro* (Fig. 4, Table 3). Interestingly, dextran sulfate could be administered an hour prior to virus with no loss of function, an observation also made *in vitro* (Dyer *et al.,* J. Virol. 71(1):191-198, 1997). In addition, infection was not limited to regenerating myofibers, which were identified by their centrally-located nuclei. Taken together, these results show that the barrier to HSV infection in adult skeletal muscle was due, at least in part, to the relative paucity of HSV receptors required for efficient infection.

**Table 3**

| Number of *lacZ*-expressing fibers in mature mouse TA muscle following gene transfer by intramuscular co-injection of treatment with G207 | |
|---|---|
| **Treatment** | **# of blue fibers** |
| G207 only | 0 |
| 3 µg/ml dextran sulfate | 76 |
| 10µg/ml dextran sulfate | 149 |
| 0.18 mg/ml collagenase type IV | 0 |
| 0.33 mg/ml collagenase type IV | 77 |
| 2 U/ml chondroitin ABC lyase | 302 |
| 4 U/ml chondroitin ABC lyase | 226 |

### HSV toxicity after systemic delivery

The toxicity of HSV in mice delivered NV1020 systemically was first assessed. Mice were administered various doses of NV1020 either by an intrasplenic route, by portal vein, or by tail vein. Morbidity and mortality were assessed every day for 28 days (Table 4). These studies demonstrated that 1 x 10⁷pfu can be delivered to mice through a variety of routes without any observable toxicity. This no-effect dose is equivalent to approximately 3.5 x 10¹⁰ pfu in humans.

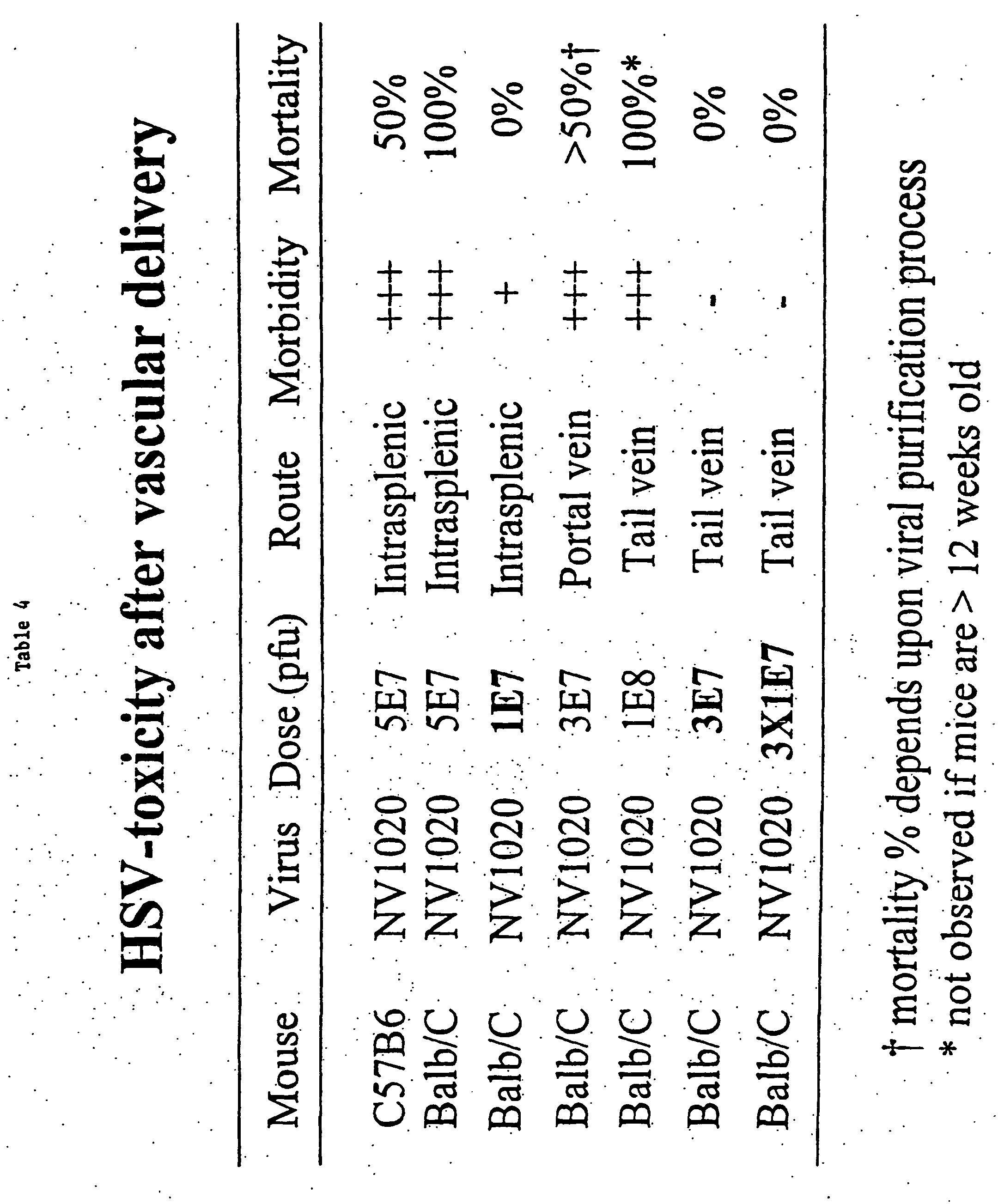

### Systemic delivery of HSV results in infection of tumor tissue

HSV can also be delivered to tumor tissue as an antitumor agent For example, HSV was administered to mice, by tail vein (G47Δ-BAC, a derivative of G207 with ICP47 deleted and a bacterial artificial chromosome (BAC) element inserted into the thymidine kinase locus), or locoregionally (G207), by portal vein, reaching a distant flank tumor or liver tumor, respectively, The virus successfully infected the tumor cells in each of the models (Fig. 5). Viral induced destruction of tumor cells can be followed by tumor necrosis, resulting in delayed tumor growth and regression.

### Delivery of viral particles to various tissues after systemic administration

The estimated number of viral particles that reach a tumor after systemic administration was also determined. Mice with flank tumors were injected, by tail vein, with 1 x 10⁷ pfu of ³⁵S methionine-labeled NV1020. NV1020 is a recombinant replication competent vector containing only one copy of γ34.5 and a deletion in the terminal repeats such that rearrangement of genome segments is ablated. After 2 hours, the mice were sacrificed, and their tissues were harvested and measured for the presence of virus particles (by measuring the radioactive label). The results of these studies indicated that approximately 10³ to 10⁴ viral particles were found in the liver, and approximately 10⁴ to 10⁵ particles were located in the tumor tissue (Fïg. 6).

### Systemic delivery of NV1020 significantly increases survival in a CT-26 liver metastatic cancer model

NV 1020 was also used to examine the effect of administration of this viral vector on mice in a metastatic cancer model. In this model, liver tumor nodules formed in mice following an intrasplenic injection of CT-26 cancer cells. Twenty-four hours after cell injection the mice were injected, by tail vein, with 1 x 10⁷ pfu of NV1020. The mice were carefully monitored and assessed for survival over time (Fig. 7). Moribund animals were appropriately sacrificed. Survival was increased from 25% to 75% by the administration of NV1020, compared to control animals.

### Intratumoral of systemic delivery of NV1020 results in anti-tumor efficacy in a CT-26 flank tumor model

Different modes of delivery were also examined to determine the efficacy of systemic delivery of HSV on tumor growth. Flank tumors were established in mice by subcutaneous injection of CT-26 cancer cells. The mice were then administered 1 x 10⁷ pfu of NV 1020 either intratumorally or systemically by tail vein. Tumor growth rates were then assessed by measuring the tumor volume biweekly (Fig. 8).

The results of this study indicate that intratumoial administration of NV1020 is as efficacious as systemic delivery by tail vein (no statistical difference). Both routes of administration significantly delayed the tumor growth rate, resulting in an approximately 50% reduction in tumor volume. These results demonstrate that although virus is typically administered by intratumoral injection in tumor models (because it is thought that systemic delivery of virus would not result in sufficient pfu reaching the tumor for efficacy), other routes of administration are also effective.

The addition of a glycosaminoglycan analog to the viral vector therapy for tumor treatment was also investigated, Dextran sulfate is a glycosaminoglycan analog that is used in a research setting to block viral infection of target cells by interfering with viral attachment to cells. In a clinical setting, it is used for local perfusion of therapeutics following surgery. Dextran sulfate is also reported to be a volume expander. Most recently, dextran sulfate has been tested as an antiviral agent for HIV.

The CT-26 flank tumor model was again used to determine the effect of a combination of HSV and dextran sulfate analog on tumor growth. A combination of 1 x 10⁷ pfu of NV1020 and dextran sulfate (100 µg/ml) was administered, by tail vein, to mice with flank tumors, and the tumor volume was assessed over time. NV1020 plus dextran decreased the tumor volume as well as NV1020 alone (Fig. 8).

### Anti-tumor efficacy increases with dose of dextran sulfate administered

The effect of the concentration of charged molecule administered with HSV on tumor growth was also evaluated. Mice with flank tumors were administered one dose of 1 x 10⁷ pfu of NV1020,1 x 10⁷ pfu of NV1020 plus 10 µg/ml of dextran sulfate, 1 x 10⁷ pfu of NV1020 plus 100 µg/ml of dextran sulfate (100 µg/ml), 1 x 10⁷ pfu of NV1020 plus 500 µg/ml of dextran sulfate, or PBS plus 100 µg/ml of dextran sulfate (control) at day 0, day 2, and day 4. Tumor growth rates were then assessed (Fig. 9). These studies revealed that dextran sulfate enhanced viral therapy in a dose-dependent manner, with higher concentrations of dextran sulfate co-administered with NV1020 being more efficacious than lower concentrations.

### Multiple-dosing increases anti-tumor efficacy in a CT-26 flank tumor model

The effect of multiple doses of HSV plus dextran sulfate on tumor growth was also evaluated. Mice with flank tumors were administered three doses of 1 x 10⁷ pfu of NV1020, one dose of 3 x 10⁷ pfu of NV1020, three doses of 1 x 10⁷ pfu of NV1020 plus 100 µg/ml of dextran sulfate (F1), or PBS (control). Tumor growth rates were then assessed (Fig. 10).

The results of this study showed that tumor growth was lower when 100 µg/ml of dextran sulfate was co-injected with NV1020. In addition, anti-tumor efficacy is increased when three doses of 1 x 10⁷ pfu of NV1020 were administered compared to when one dose of 3 x 10⁷ pfu of NV1020 was administered.

### Multiple dosing of NV1020 with dextran sulfate increases survival in a CT-26 flank tumor model

Many reports of anti-tumor efficacy do not result in a corresponding increase in subject survival. To determine if anti-tumor efficacy corresponds to an increase in survival in animals treated with HSV or HSV plus dextran sulfate, mice with flank tumors were administered one dose of 3 x 10⁷ pfu of NV1020, one dose of 3 x 10⁷ pfu of NV1020 plus 100 µg/ml of dextran sulfate, three doses of 1 x 10⁷ pfu of NV1020 plus 100 µg/ml of dextran sulfate, or PBS (control). Mouse survival was then measured over time (Fig. 11). These studies showed that NV1020, delivered in multiple doses along with dextran sulfate, increased the survival of mice. These results correspond to the anti-tumor efficacy of NV1020 plus dextran sulfate described above. With such a treatment, cures can result.

### Dextran sulfate increases efficacy in a CT-26 liver metastatic model

The efficacy of formulation changes were also evaluated in a mouse CT-26 liver metastatic model. Mice with metastasized tumors were administered 1 x 10⁷ pfu of NV1020, 1 x 10⁷ pfu of NV1020 plus dextran sulfate (100 µg/ml), PBS only (control), or PBS plus dextran sulfate (100 µg/ml)(control). The number of tumor nodules were then assessed 13 days after treatment (Fig. 12). Treatment with each of the three formulations resulted in decreased nodule counts compared to controls. These results establish that this treatment protocol is not only effective in the instance of single, large, established flank tumors, but also in the instance of microscopic disease.

### Dextran and acyclovir also increase anti-tumor efficacy in a CT-26 an tumor model

To examine the roles of sulfation of dextran sulfate and the replication of HSV in the anti-tumor efficae of HSV plus dextran sulfate formulations, dextran or acyclovir were added to the formulations. The molecule dextran has been used in the clinic as a volume expander and for local perfusion of tissue following surgery. There are also reports of using dextran for routine cardiovascular therapy in Japan. Acyclovir, is a clinically approved drug, used to prevent replication and hence inhibit the spread of HSV. It is an obligatory chain terminator activated by viral thymidine kinase.

Mice with flank tumors were administered 1 x 10⁷ pfu of NV1020, 1 x 10⁷ pfu of NV1020 plus dextran sulfate (100 µg/ml), 1 x 10⁷pfu of NV1020 plus dextran only, 1 x10⁷ pfu of NV1020 plus dextran sulfate (100 µg/ml) and acyclovir (2 mg/ml), or PBS plus dextran sulfate (100 µg/ml). Tumor growth rates, were then assessed biweekly (Fig. 13). The sulfate component of dextran sulfate did not appear to affect its mode of increasing anti-tumor efficacy, as co-injection of HSV with dextran alone gave a comparable anti-tumor efficacy. As well, viral replication does not appear to be necessary for anti-tuimor efficacy or mouse survival, as the combination of HSV, dextran sulfate, and acyclovir reduced tumor growth.

### G207 anti-tumor efficacy is enhanced by dextran sulfate

Another recombinant HSV vector, G207, a different strain than NV1020 with both copies of γ34.5 deleted and inactivation of ribonucleotide reductase by insertion of the β-galactosidase gene was also tested for its anti-tumor efficacy. Mice with flank tumors were administered 1 x 10⁷ pfu of NV1020, 1 x 10⁷ pfu of G207, 1 x 10⁷ pfu of G207 plus dextran sulfate (100 µg/ml), or PBS plus dextran sulfate (100 µg/ml)(control) at day 0, day 2, and day 4. Tumor growth rates were then measured (Fig. 14). Dextran sulfate increased anti-tumor efficacy of G207, indicating that other strains of HSV vectors are of therapeutic value, and that HSV-1 anti-tumor efficacy is not dependent on γ34.5. These findings also suggest that other current generation vectors may also be used therapeutically.

### Dextran sulfate alters CT-26 -morphology in vitro, but not cell growth

To better understand the *in vivo* effects of dextran sulfate or dextran in combination with HSV, the effects of dextran sulfate on cell morphology and cell proliferation were evaluated in a cell culture model. CT-26 cells were treated with 100 µg/ml of dextran sulfate or were left untreated. After 48 hours, cell numbers and morphology were examined (Fig. 15). *In vitro,* dextran sulfate added to cell culture media did not slow the growth of CT-26 cells. Treatment of the cells with dextran sulfate did, however, change the morphology of the cells. In the presence, of dextran sulfate, CT-26 cells appeared more evenly spread across tissue culture dishes (panel B) as compared to the "clumped" appearance of CT-26 cells in the absence of dextran sulfate (panel A). These results suggest that a change in gene expression may account for the profound anti-tumor efficacy seen *in vivo*.

### Dextran sulfate and acyclovir do not affect-CT-26 growth in culture

The effect of dextran sulfate, acyclovir, or a combination of both dextran and acyclovir were also examined for their effects on cell growth *in vitro.* Cultured CT-26 cells were administered dextran sulfate (100 µg/ml), acyclovir (2 mg/ml), both dextran sulfate and acyclovir, or were left untreated (control). CT-26 cells were counted at various time points following addition each formulation. After 72 hours of exposure to the formulations, the growth of the cells (number of cells/ml) did not vary significantly upon exposure to dextran sulfate, acyclovir, or a combination of both formulations, compared to untreated cells (Fig. 16). Such results indicate that these formulations do not alter cell growth *in vitro.*

### Dextran sulfate increases peripheral degeneration of tumors

The result of HSV co-injected with dextran sulfate on tumor degeneration was examined next. Mice with flank tumors were administered. NV1020 or NV1020 plus dextran sulfate (F1) by tail vein. The tumor was then removed, frozen, and sectioned for histochemical analysis (Fig. 17). Peripheral tumor degeneration was much greater when dextran sulfate was administered with NV1020 (panel B; area shown in light grey), than when NV1020 was administered alone (panel A). This indicates that tumor necrosis is not limited to anoxic cells at the center of the tumor, but also occurs at growing and dividing tumor cells located at the margin where blood vessels feeding the tumor are located.

### Dextran sulfate increases the bioavailability of virus in vivo

As it is known that virus is quickly inactivated by components present in blood, including those of the immune system (complement factors, antibodies), the effect of dextran sulfate on the bioavailability of virus *in vivo* was examined. One x 10⁷ pfu of NV1020 or 1 x 10⁷ pfu of NV1020 plus dextran sulfate (100 µg/ml) was administered by tail vein to mice. Groups of mice were then sacrificed at varying time points. Blood samples were removed by heart puncture, and serum was analyzed to determine the length of time in which the virus was infective *in vivo* (Fig. 18). When dextran sulfate was administered with NV1020, the circulation time of infectious virus was increased by 3-fold. Such a time is sufficient to enable the active virus to reach a tumor in order to mediate anti-tumor efficacy. Accordingly, a longer circulation time of infectious virus results in a greater net therapeutic vector delivered.

### Dextran sulfate alters the in vivo distribution of NV1020 such that more virus reaches tumor tissue

In addition, the effect of dextran sulfate on the *in vivo* tissue distribution of NV1 020 was determined. One x 10⁷ pfu of radioactive-labeled (³⁵S) NV1020 or 1 x 10⁷ pfu of radioactive-labeled (³⁵S) NV1020 with dextan sulfate (100 µg/ml) was administered to mice with flank tumors by tail vein. At 2 hours or 12 hours; various organs, including liver, spleen, kidney, lung, and heart, as well as the tumor were harvested, homogenized, and assessed for viral load by scintillation counting (Fig. 19). Over time, dextran sulfate increased the amount of virus found in the tumor and decreased the amount of virus found in the liver.

### Dextran sulfate decreases angiopoiesis factor gene expression

Gene expression studies in CT 26 cells treated with dextran sulfate were also completed. Cultured CT-26 cells were treated for 1 hour with dextran sulfate, or were left untreated. RNA was then extracted from the cells. Expression of a number of different genes was then measured, and the expression levels between the cells treated with dextran sulfate and untreated cells was compared. The results of these studies is summarized in Table 5. Notably, expression of a number of genes encoding proteins involved in angiopoiesis were decreased.

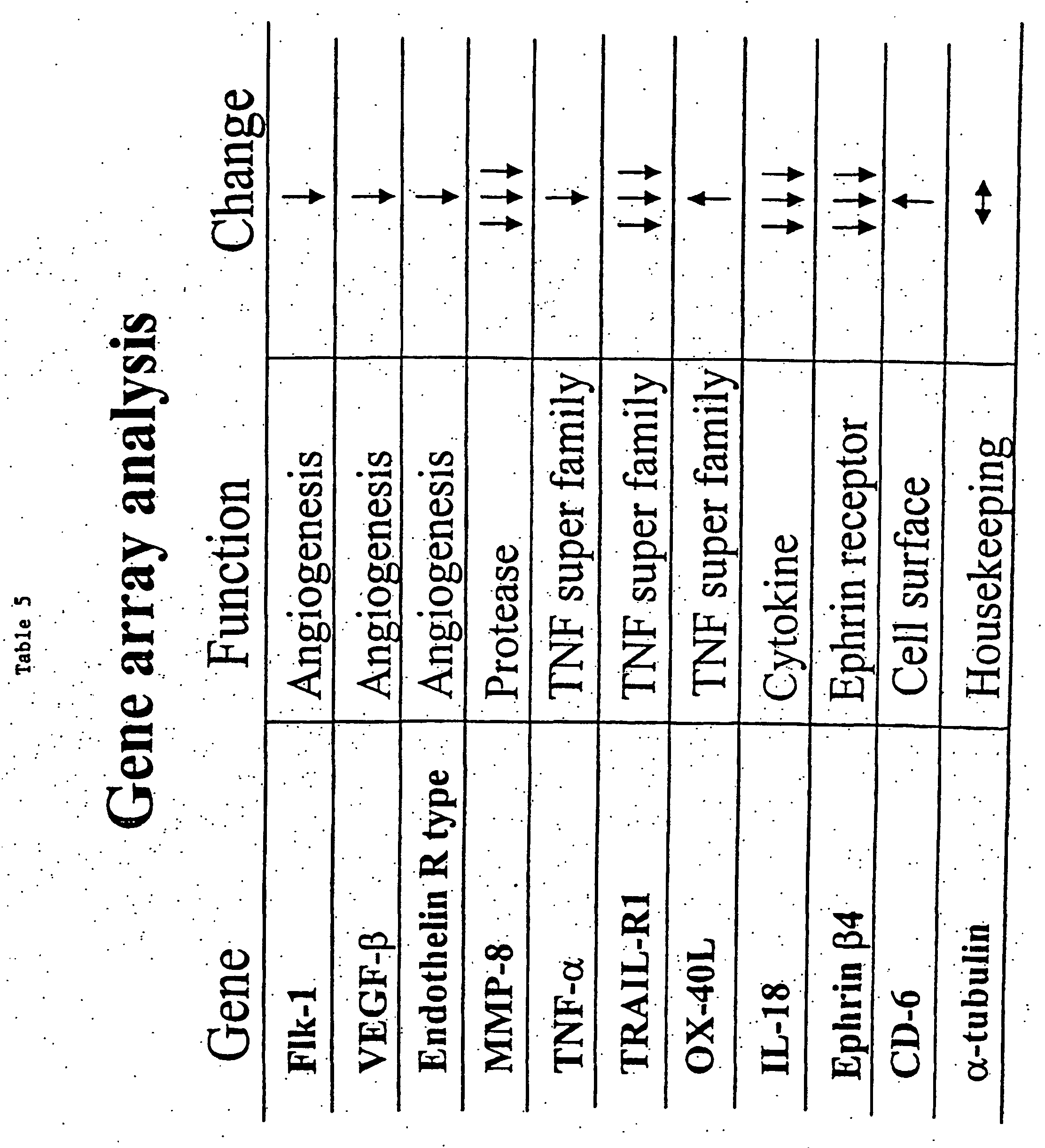

### Materials and Methods

### Materials

Dextran sulfate with a molecular weight of 500,000 was purchased from Pharmacia (catalog no. 17-0340-01). Chondroitin ABC lyase was purchased from Seikagaku Corporation (catalog no. 100330). Heparitinase was purchased from Seikagaku Corporation (catalog no. 100703). Collagenase type IV was purchased from Sigma (catalog no. C 1889). All tissue culture reagents (Gibco) and dishes (Nunc) were obtained from Canadian Life Technologies (Burlington, Ontario, Canada).

### Viral stocks

Recombinant NV 1020, HSV-1, G207 (NeuroVir Inc.) and HSV-2, L1BR1 (Asano *et al.,* J. Gen. Virol. 80(1):51-56, 1999; Nishiyama *et al.,* Virology 190(1):256-268, 1992) were prepared on Vero cells. The recombinant HSV vector, NV1020, a replication competent vector contains only one copy of γ34.5 and has a deletion in the terminal repeats such that rearrangement of genome segments is ablated. G207 contains the β-galactosidase gene inserted in-frame in the ribonucleotide reductase gene. As such, this recombinant virus is unable to replicate in non-dividing cells (*e.g.*, muscle cells). L1BR1 contains the β-galactosidase gene inserted into the US3 protein kinase gene. Virus was concentrated by centrifugation through a 30% sucrose pad, suspended in phosphate buffered saline (PBS), and filtered through a 0.45 µm filter (Sartorius), using standard methods. The final titer of infectious virus used for all experiments was 1 x 10⁸ pfu/ml.

### Primary muscle fiber cultures

Balb/c mice were bred in institutional animal care facilities at the University of British Columbia. Two different age groups were designated to be "newborn" and "adult." The "newborn" mice were 7 to 10 days old. The "adult" mice were 6 to 12 weeks old.

Single isolated myofibers were prepared from dissected extensor digitorum longus (EDL) muscle. The myofibers were dissociated by enzymatic disaggregation in 0.2% type 1 collagenase (Sigma), followed by mild trituration. Isolated myofibers were then plated into several 24 well dishes coated with 1 mg/ml of Matrigel (Collaborative Biomedical Products). Culture medium consisting of 10% horse serum and 10% FBS in DMEM was added to the wells. These plates were then incubated for 18 hours at 37°C, at which point viable myofibers were infected with G207.

### Infection of myofibers

Myofibers were infected by adding G207 (10⁶ pfu) in culture medium (10% FBS in DMEM) directly to the wells. Incubation length was overnight (approximately 18 hours), although a one hour infection in DMEM only was sufficient to give reproducible infection. Following incubation, myofibers were fixed for 15 minutes in 1.25% glutaraldehyde and stained with 2% X-gal substrate (1 mM MgCl₂, 5 mM K₄Fe(CN)₆/K₃Fe(CN)₆ in PBS) (Canadian Life Technologies) for 4 hours at 37°C.

### Indirect immunonofluorescence

Isolated myofibers were plated onto glass coverslips and infected with G207 as described above. They were then fixed in 1.25% glutaraldehyde in PBS for 15 minutes, rinsed twice with PBS, followed by 15 minutes incubation in the blocking solution (PBS with 1% bovine serum albumin (Boehringer Mannheim)). After blocking, myofibers were permeabilized with 0.1 % Triton-X100/PBS for 5 minutes and incubated with a mouse anti-ICP4 antibody at 1:2000 for 1 hour. Myofibers were washed with three changes of PBS, then incubated with goat anti-mouse IgG conjugated to Texas-Red (Jackson Immunochemicals) diluted 1:200 in PBS-1 % BSA for 30 minutes. The myofibers were then rinsed with PBS and mounted on glass slides. Immunofluorescence staining was observed using a BioRad MRC 600 confocal epifluorescence microscope. Confocal images were rendered using NIH Image Version 1.60 and colorized with Adobe Photoshop Version 4.0 (Adobe Systems Inc.). Standard control experiments were performed, including incubation with the secondary antibody only and with mock infected cells. All fixation and antibody incubations were performed at RT.

### In vitro treatment assays

Assays for dextran sulfate stimulation, collagenase type IV, chondroitin ABC lyase, and heparitinase were performed on adult myofibers plated in 24 well dishes. The myofibers were pretreated with varying concentrations of dextran sulfate, collagenase type IV, chondroitin ABC lyase, or heparitinase in DMEM for 30 minutes prior to infection. After an overnight adsorption period (approximately 18 hours) at 37°C, the inoculum was removed. The myofibers were then fixed for 15 minutes in 1.25% glutaraldehyde and stained with 2% X-gal substrate for 4 hours at 37°C. For all *in vitro* studies, a minimum of 40 myofibers were tested per treatment group unless otherwise specified. PEG-induced fusion was performed according to methods described previously (Meyer *et al.,* J. Gen. Virol. 79(8):1983-1987, 1998).

### Analysis of glycosaminoglycans

Biochemical labeling of glycosaminoglycans was performed by a modification of procedures described previously (Bame *et al.,* J. Biol. Chem. 264:8059-8065, 1989). Briefly, glycosaminoglycans were radiolabeled by incubating cells for 24 hours with [³⁵S] sulfate (carrier free, approximately 43 Ci/mg, ICN) per ml in DMEM/10% FBS/10% horse serum modified to contain 10 µM sulfate. The cells were washed three times with cold PBS and solubilized with 1 ml of 0.1 N NaOH at RT for 15 minutes. Samples were removed for protein determination. Extracts were adjusted to pH 5.5 by the addition of concentrated acetic acid and treated with protease (Sigma; 2 mg/ml) in 0.32 M NaCl 40 mM sodium acetate, pH 5.5, containing shark cartilage chondroitin sulfate (2 mg/ml) as carrier, at 40°C for 12 hours. For some experiments, portions of the radioactive material were treated for 12 hours at 40°C with 10 mU of chondroitin ABC lyase (Sigma) or 0.5 U of hepantinase (Sigma). The radioactive products were quantified by chromatography on DEAE-Sephacel (Pharmacia) by binding in 50 mM NaCl followed by elution with 1 M NaCl. For high pressure liquid chromatography (HPLC) analysis, the glycosaminoglycan samples were desalted by precipitation with ethanol. Following centrifugation, the ethanol precipitates were suspended in 20 mM Tris (pH 7.4) and resolved by anion-exchahge HPLC, using TSK DEAE-35W column (15 by 75 mm; Beckman instruments). Proteoglycans were eluted from the column by using a linear 50 to 700 mM NaCl gradient formed in 10 mM KH₂PO₄ (pH 6.0). All buffers contained 0.2% Zwittergent 3-12 (Calbiochem). The glycosaminoglycans in the peaks were identified by digestion of the sample with the relevant enzymes prior to chromatography.

### Flank tumor model

Mice were anesthetized using ketamine (70 mg/kg) and xylazine (10 mg/kg). CT-26 cells (5 x 10⁴ cells resuspended in 100 µl of PBS) were injected subcutaneously into the right flank of each mouse, using a 26-gauge needle. The cells formed a tumor which was allowed to grow to a size of approximately 100 to 150 mm³. Injections of the desired therapy were then initiated. Tumor volumes were generally measured biweekly. The animals were sacrificed once the tumor volume reached 1500 mm³.

### Metastatic cancer model

The metastatic cancer mode has been described by Lafreniere and Rosenberg (J. Natl. Cancer Inst. 76(2):309-22, 1986).

### Intramuscular administration of the recombinant HSV vector

Adult and newborn mice under anesthesia (Ketamine/Rhompun intraperitoneally) were injected percutaneously into the tibialis anterior muscle (TA) to an approximate depth of 2.0 mm using a Hamilton syringe. For *in vivo* assays involving co-injection of treatment solutions along with the viral inoculum, dextran sulfate, collagenase type IV, or chondroitin ABC lyase was diluted to the appropriate concentration (as identified by *in vitro* studies) with G207 in an injection volume of 50 µl (for adult mice) or 25 µl (for newborn mice). Control muscles were injected with G207 only. To evaluate myofiber infection, muscles were removed 3 days post-injection for sectioning and histological analysis. For any of the procedures, a minimum of 4 animals received identical treatment and comprised an experimental group.

### Tail vein administration of the recombinant HSV vector

Tail vein administration of the desired therapy was carried out using techniques commonly known in the art.

### Tissue sectioning

The injected and control muscle or tumor tissue were rapidly frozen. The muscles or tumor tissue were sectioned, yielding serial cross-sections throughout the tissue. Cross-sections (10 µm) were cut on a cryostat and stained with X-gal and/or hematoxylin and eosin. The sections were retained at regular intervals (approximately every 120 µm). For histology, the cryosections were collected onto gelatin-coated glass slides.

### Histological analysis

The histological detection of β-galactosidase-expressing cells in cryosections was done using X-gal. This compound yields a blue reaction product in cells expressing high levels of β-galactosidase. The sections were first fixed by dipping the slides in 4% paraformaldehyde in 100 mM NaP, pH 7.2, for 5 minutes. The slides were rinsed three times for 5 minutes in PBS. The sections were then stamed with X-gal (Sigma) at a concentration of 1 mg/ml in 5 mM K₃Fe(CN)₆, 5 mM K₄Fe(CN)₆, 2mM MgCl₂ in PBS for 12 hours. The slides were mounted using an aqueous mounting medium (Promount) and examined microscopically for the presence of β-galactosidase-labeled ("blue") myofibers. The total number of lacz-expressing fibers in a muscle was determined from the section with the maximal number of blue fibers, and that was invariably at the site of implantation.

Alternatively, cells infected with HSV were detected using standard immunohistochemical procedure and an antibody that recognizes HSV antigen.

### Determining the bioavailablity of virus in vivo

The serum from blood obtained from mice infected with NV1024 was applied to cultured cells. The duration of time for which the virus was able to infect the cells was determined as described above.

## Claims

1. Use of a nucleic acid vector for the preparation of a pharmaceutical composition for introducing a nucleic acid vector into a living cell, wherein said vector is a viral vector of the family Herpesviridae, and said vector is introduced into said cell by a method comprising contacting said cell with said vector and, either before, during, or after contacting said cell with said vector, contacting said cell with a liquid medium comprising a compound that, in said medium, is charged, noncytotoxic, and capable of facilitating the uptake of the vector by the cell, wherein said cell is in a mammal.

2. The use of claim 1, wherein said mammal is a human patient.

3. The use of claim 1, wherein said vector comprises a gene encoding a polypeptide, a hormone, a vaccine antigen, an antisense molecule, or a ribozyme.

4. The use of claim 3, wherein said polypeptide is selected from the group consisting of growth factors, enzymes, anti-angiogenic polypeptides, and polypeptides that promote cell death.

5. The use of claim 1, wherein said vector is selected from the group consisting of HSV-1, HSV-2, VZV, CMV, EBV, HHV-6, HHV-7, and HHV-8.

6. The use of claim 1, wherein said vector is attenuated.

7. The use of claim 1, wherein said charged molecule is selected from the group consisting of charged polysaccharides, polylysine, acyclodextrin, diethylaminoethane, and polyethylene glycol.

8. The use of claim 7, wherein said charged polysaccharide is a glycosaminoglycan.

9. The use of claim 7, wherein said charged polysaccharide is a glycosaminoglycan analog.

10. The use of claim 8, wherein said glycosaminoglycan is selected from the group consisting of dermatan sulfate, heparan sulfate, chondroitin sulfate, and keratin sulfate.

11. The use of claim 9, wherein said glycosaminoglycan analog is dextran sulfate.

12. The use of claim 1, wherein said charged molecule is administered to said cell prior to the administration of said vector to said cell.

13. The use of claim 1, wherein said charged molecule is administered to said cell concurrent with the administration of said vector to said cell.

14. The use of claim 1, wherein said cell is a mature muscle cell.

15. The use of claim 2, wherein said cell is a cancer cell.

16. The use of claim 15, wherein said patient has cancer.

17. The use of claim 14, wherein said muscle cell is in a patient with a primary myopathy.

18. The use of claim 2, wherein said patient has a condition that can be treated by production of a therapeutic product for secretion into said subject's circulation.

19. The use of claim 2, wherein said vector and charged molecule are delivered locally.

20. The use of claim 2, wherein said vector and charged molecule are delivered systemically.

## Patentansprüche

1. Verwendung eines Nukleinsäurevektors zur Herstellung einer pharmazeutischen Zusammensetzung zum Einführen eines Nukleinsäurevektors in eine lebende Zelle, wobei der Vektor ein viraler Vektor der Familie Herpesviridae ist und der Vektor in die Zelle durch ein Verfahren eingeführt wird, das umfasst das Inkontaktbringen der Zelle mit dem Vektor und entweder vor, während oder nach dem Inkontaktbringen der Zelle mit dem Vektor das Inkontaktbringen der Zelle mit einem flüssigen Medium, das eine Verbindung enthält, die in dem Medium geladen, nicht toxisch und in der Lage ist, die Aufnahme des Vektors durch die Zelle zu erleichtern, wobei die Zelle in einem Säuger ist.

2. Verwendung nach Anspruch 1, wobei der Säuger ein menschlicher Patient ist.

3. Verwendung nach Anspruch 1, wobei der Vektor ein Gen umfasst, das ein Polypeptid, ein Hormon, ein Impfstoffantigen, ein Antisense-Molekül oder ein Ribozym kodiert.

4. Verwendung nach Anspruch 3, wobei das Polypeptid ausgewählt ist aus der Gruppe bestehend aus Wachstumsfaktoren, Enzymen, anti-angiogenen Polypeptiden und Polypeptiden, die den Zelltod vermitteln.

5. Verwendung nach Anspruch 1, wobei der Vektor ausgewählt ist aus der Gruppe bestehend aus HSV-1, HSV-2, VZV, CMV, EBV, HHV-6, HHV-7 und HHV-8.

6. Verwendung nach Anspruch 1, wobei der Vektor attenuiert ist.

7. Verwendung nach Anspruch 1, wobei das geladene Molekül ausgewählt ist aus der Gruppe bestehend aus geladenen Polysacchariden, Polylysin, Acyclodextrin, Diethylaminoethan und Polyethylenglykol.

8. Verwendung nach Anspruch 7, wobei das geladene Polysaccharid ein Glucosaminoglycan ist.

9. Verwendung nach Anspruch 7, wobei das geladene Polysaccharid ein Glucosaminoglycananalog ist.

10. Verwendung nach Anspruch 8, wobei das Glucosaminoglycan ausgewählt ist aus der Gruppe bestehend aus Dermatansulfat, Heparansulfat, Chondroitinsulfat und Keratinsulfat.

11. Verwendung nach Anspruch 9, wobei das Glucosaminoglycananalog Dextransulfat ist.

12. Verwendung nach Anspruch 1, wobei das geladene Molekül der Zelle vor der Verabreichung des Vektors an die Zelle verabreicht wird.

13. Verwendung nach Anspruch 1, wobei das geladene Molekül der Zelle gleichzeitig mit der Verabreichung des Vektors an die Zelle verabreicht wird.

14. Verwendung nach Anspruch 1, wobei die Zelle eine reife Muskelzelle ist.

15. Verwendung nach Anspruch 2, wobei die Zelle eine Krebszelle ist.

16. Verwendung nach Anspruch 15, wobei der Patient Krebs hat.

17. Verwendung nach Anspruch 14, wobei die Muskelzelle in einem Patienten mit einer primären Myopathie ist.

18. Verwendung nach Anspruch 2, wobei der Patient einen Zustand hat, der durch die Produktion eines therapeutischen Produkts für die Sekretion in den Blutkreislauf des Subjekts behandelt werden kann.

19. Verwendung nach Anspruch 2, wobei der Vektor und das geladene Molekül lokal übertragen (delivered) werden.

20. Verwendung nach Anspruch 2, wobei der Vektor und das geladene Molekül systemisch übertragen werden.

## Revendications

1. Utilisation d'un vecteur d'acide nucléique pour la préparation d'une composition pharmaceutique en vue d'introduire un vecteur d'acide nucléique dans une cellule vivante, lequel vecteur est un vecteur viral de la famille des *Herpesviridae,* et ledit vecteur est introduit dans ladite cellule par une méthode comprenant le contact de ladite cellule avec ledit vecteur et, soit avant, soit durant, soit après un contact de ladite cellule avec ledit vecteur, le contact de ladite cellule avec un milieu liquide comprenant un composé qui, dans ledit milieu, est chargé, non cytotoxique et susceptible de faciliter la reprise du vecteur par la cellule, ladite cellule étant celle d'un mammifère.

2. L'utilisation selon la revendication 1, dans laquelle ledit mammifère est un patient consistant en un être humain.

3. L'utilisation selon la revendication 1, dans laquelle ledit vecteur comprend un gène codant pour UN polypeptide, une hormone, un antigène de vaccin, une molécule antisens ou un ribozyme.

4. L'utilisation selon la revendication 3, dans laquelle ledit polypeptide est choisi dans le groupe comprenant des facteurs de croissance, des enzymes, des polypeptides anti-angiogènes et des polypeptides qui promulguent la mort des cellules.

5. L'utilisation selon la revendication 1, dans laquelle ledit vecteur est choisi dans le groupe comprenant HSV-1 HSV-2, VZV, CMV, EBV, HHV-6, HHV-7 et HVV-8.

6. L'utilisation selon la revendication 1, dans laquelle ledit vecteur est atténué.

7. L'utilisation selon la revendication 1, dans laquelle la molécule chargée est choisie dans le groupe comprenant des polyéthylènes glycol, diéthyl-aminoéthane, acyclodextrine, polysine, et polysaccharide chargés.

8. L'utilisation selon la revendication 7, dans laquelle le polysaccharide chargé est une glycosaminoglycane.

9. L'utilisation selon la revendication 7, dans laquelle le polysaccharide chargé est un analogue de la glycosaminoglycane.

10. L'utilisation selon la revendication 8, dans laquelle ladite glycosaminoglycane est choisie dans le groupe comprenant sulfate de dermatane, sulfate d'héparane, sulfate de chondroïtine, et sulfate de kératine.

11. L'utilisation selon la revendication 8, dans laquelle ledit analogue de la glycosaminoglycane est du sulfate de dextrane.

12. L'utilisation selon la revendication 1, dans laquelle ladite molécule chargée est administrée à ladite cellule préalablement à l'administration dudit vecteur de ladite cellule.

13. L'utilisation selon la revendication 1, dans laquelle ladite molécule chargée est administrée à ladite cellule en même temps que l'administration dudit vecteur à ladite cellule.

14. L'utilisation selon la revendication 1, dans laquelle ladite cellule est une cellule de muscle mature.

15. L'utilisation selon la revendication 2, dans laquelle ladite cellule est une cellule cancéreuse.

16. L'utilisation selon la revendication 15, dans laquelle ledit patient est atteint du cancer.

17. L'utilisation selon la revendication 14, dans laquelle la cellule musculaire est celle d'un patient présentant une myopathie primaire.

18. L'utilisation selon la revendication 2, dans laquelle ledit patient présente une maladie qui doit être traitée par production d'un produit thérapeutique destiné à être secrété dans la circulation dudit patient.

19. L'utilisation selon la revendication 2, dans laquelle ledit vecteur et la molécule chargée sont administrés localement.

20. L'utilisation selon la revendication 2, dans laquelle ledit vecteur et la molécule chargée sont administrés par voie systémique.
